(19) Europäisches Patentamt
European Patent Office
Office européen des brevets

(11) Publication number: 0 308 020 A2

# (12) EUROPEAN PATENT APPLICATION

(21) Application number: 88201974.8

(22) Date of filing: 09.09.88

(51) Int. Cl.[4]: C07D 213/80 , C07D 409/14 , A61K 31/44

(30) Priority: 18.09.87 US 98632

(43) Date of publication of application: 22.03.89 Bulletin 89/12

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Biftu, Tesfaye
25 Victorian Drive
Old Bridge New Jersey 08857(US)
Inventor: Heck, James V.
961 Nepawin Lane
Scotch Plains New Jersey 07076(US)
Inventor: Thorsett, Eugene D.
4 Poplar Place
Fanwood New Jersey 07023(US)

(74) Representative: Hesketh, Alan, Dr.
European Patent Department Merck & Co., Inc. Terlings Park Eastwick Road
Harlow Essex, CM20 2QR(GB)

## (54) 5-(aryl and heteroaryl)-6-(aryl and heteroaryl)-1,2-dihydro-2-oxo 3-pyridinecarboxylic acids and derivatives thereof.

(57) This invention relates to a method of treating bacterial infections with 5-(aryl and heteroaryl)-6-(aryl and heteroaryl)-1,2-dihydro-2-oxo-3-pyridinecarboxylic acids and derivatives thereof. This invention further relates to pharmaceutical compositions that are useful in the treatment of bacterial infections.

EP 0 308 020 A2

# 5-(ARYL AND HETEROARYL)-6-(ARYL AND HETEROARYL)-1,2-DIHYDRO-2-OXO-3-PYRIDINECARBOXYLIC ACIDS AND DERIVATIVES THEREOF

## BACKGROUND OF THE INVENTION

### (a) Field of the Invention

This invention relates to certain 5-(aryl and heteroaryl)-6-(aryl and heteroaryl)-1,2-dihydro-2-oxo-3-pyridinecarboxylic acids and derivatives thereof that are useful in the treatment of bacterial infections. In particular, this invention relates to a method of treating bacterial infections with said compounds. This invention further relates to pharmaceutical compositions containing said compounds and to a method of treating bacterial infections with said pharmaceutical compositions.

A multitude of broad-spectrum antibiotics useful in the treatment of both Gram-positive and Gram-negative bacterial infections, as well as other microbial infections, are known. However, a continuing need for new antibiotics exists. For example, known antibiotics may be effective against only certain strains of microorganisms. Furthermore, and perhaps more seriously, continued widespread use can give rise to resistant strains of microorganisms against which a particular antibiotic or group of antibiotics was previously effective. The compounds of this invention were discovered as part of a search for new antibiotics intended to overcome such problems.

### (b) Prior Art

Certain 1,2-dihydro-2-oxo-3-pyridinecarboxylic acid derivatives are known, including various 5,6-disubstituted derivatives. For example, Adachi, Chem. Pharm. Bull., 17, 2209-2216 (1969), discloses, inter alia, the preparation of 5,6-diphenyl-1,2-dihydro-2-oxo-3-pyridinecarboxylate esters but fails to describe any biological activity.

U.S. Patent 3,655,679 discloses 5-alkyl-6-phenyl and 6-alkyl-5-phenyl derivatives of 1,2-dihydro-2-oxo-3-pyridinecarboxylic acid but does not disclose nor suggest 5,6-diphenyl substitution. Moreover, the '679 patent discloses only antiinflammatory, antipyretic, and analgesic activities, and fails even to suggest antibiotic activity.

Japanese Patent 85-94964 discloses certain 6-aryl-5-(substituted pyridyl) derivatives having activity as cardiac stimulants acting on the papillary muscle. The Japanese patent neither discloses nor suggests antibacterial activity.

Honma et al., Chem. Pharm. Bull., 30, 4314-4324 (1982), discloses 6-phenyl derivatives of 1,2-dihydro-2-oxo-3-pyridinecarboxylic acid that exhibit antiallergy activity. The reference neither discloses nor suggests 5,6-disubstitution or antibacterial activity.

U.S. Patents 3,928,366, 3,838,156, and 3,838,120 discloses certain 6-(3- or 4-pyridyl) derivatives of 1,2-dihydro-2-oxo-3-pyridinecarboxylates as intermediates used to prepare antibacterial 2-(substituted amino)-3-pyridinecarboxylates. The cited patents neither disclose nor suggest 5,6-disubstitution or antibacterial activity for the 2-oxo intermediates.

Certain 1,2-dihydro-2-oxo-3-pyridinecarboxylates for which no biological properties were disclosed have also been described. See, for example, Mosti et al., J. Heterocyclic Chem., 22, 1503-1509 (1985) (5-acyl-6-phenyl); Oostveen and van der Plas, Recl. Trav. Chim. Pays-Bas, 93, 223-236 (1974) (5- or 6-phenyl); and Leaver and Vass, J. Chem. Soc., 1629-1636 (1965) (5-carboxy-6-carboxymethyl). The compounds in the cited references are structurally distinct from the compounds of the present invention.

Certain other 6-substituted and 5,6-disubstituted derivatives of 1,2-dihydro-2-oxo-3-pyridinecarboxylic acid have been coupled with various penicillins, cephalosporins, and other such antibiotics. See, for example, U.S. Patents 4,315,014, 4,278,681, 4,101,661, 3,954,734, 3,951,982, 3,948,903, 3,946,015, 3,873,523, and 3,838,120; British Patent 1,464,525; European Application 15,773; Showalter et al., J. Heterocyclic Chem., 18, 1609-1612 (1981); and Kaltenbronn et al., J. Antibiotics, 32, 621-625 (1979). The cited references, however, describe antibiotic activity for the coupled derivatives and neither disclose nor suggest that the pyridinecarboxylate moieties independently have antibiotic properties. Thus, the compounds of the present invention are both structurally and biologically distinguishable.

SUMMARY OF THE INVENTION

This invention relates to certain 5,6-disubstituted 1,2-dihydro-2-oxo-3-pyridinecarboxylic acids and derivatives thereof (also referred to as substituted 1,2-dihydro-2-oxonicotinic acids) that are useful in the treatment of bacterial infections. In particular, this invention relates to a method of treating bacterial infections with compounds of Formula I. This invention further relates to pharmaceutical compositions containing said compounds of Formula I and to a method of treating bacterial infections with said pharmaceutical compositions.

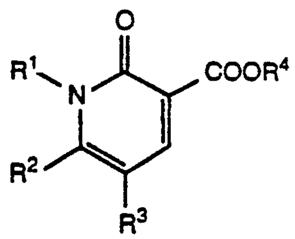

I

wherein $R^1$ is:

a) hydrogen;
b) $C_1$-$C_6$ alkyl;
c) $C_1$-$C_4$ haloalkyl;
d) $C_2$-$C_6$ alkenyl;
e) $C_4$-$C_{11}$ (cycloalkyl)alkyl; or
f) $C_7$-$C_{14}$ aralkyl;

$R^2$ and $R^3$ are independently:

a) aryl having 6, 10, or 14 nuclear ring carbon atoms or said aryl substituted with one or more substituents selected from the group consisting of:

i) halogen;
ii) hydroxy;
iii) $C_1$-$C_6$alkyl;
iv) $C_1$-$C_6$alkoxy;
v)

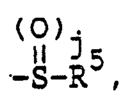

wherein $R^5$ is $C_1$-$C_6$ alkyl, and wherein j is 0, 1, or 2;

vi)

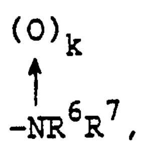

or a pharmaceutically acceptable acid addition salt thereof, wherein $R^6$ and $R^7$ are independently hydrogen or $C_1$-$C_6$ alkyl, and wherein k is 0 or 1;

vii)

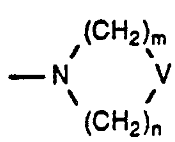

or a pharmaceutically acceptable acid addition salt thereof, wherein V is $CH_2$, O, S, SO, $SO_2$, or $NR^8$,

wherein $R^8$ is hydrogen or $C_1$-$C_6$ alkyl, and wherein m and n are independently integers of from 1 to 3;

viii) $-O(CH_2)_pNR^9R^{10}$, or a pharmaceutically acceptable acid addition salt thereof, wherein $R^9$ and $R^{10}$ are independently $C_1$-$C_6$ alkyl or taken together are $C_2$-$C_6$ alkylene, and wherein p is an integer of from 1 to 6; and

ix) -Y-$Ar^1$, wherein Y is absent or is $CH_2$, O, $-OCH_2-$, or S, and wherein $Ar^1$ is aryl having 6, 10, or 14 nuclear ring carbon atoms or is heteroaryl having 5 or 6 nuclear ring atoms of which at least one nuclear ring atom is O, S, or N; or

b) heteroaryl having 5 or 6 nuclear ring atoms of which at least one nuclear ring atom is O, S, or N, or said heteroaryl substituted with one or more substituents selected from the group consisting of:

i) halogen;

ii) hydroxy;

iii) $C_1$-$C_6$alkyl;

iv) $C_1$-$C_6$alkoxy;

v)

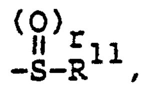

wherein $R^{11}$ is $C_1$-$C_6$ alkyl, and wherein r is 0, 1, or 2;

vi)

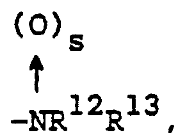

or a pharmaceutically acceptable acid addition salt thereof, wherein $R^{12}$ and $R^{13}$ are independently hydrogen or $C_1$-$C_6$ alkyl, and wherein s is 0 or 1;

vii)

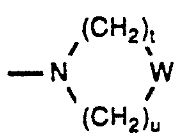

or a pharmaceutically acceptable acid addition salt thereof, wherein W is $CH_2$, O, S, SO, $SO_2$, or $NR^{14}$, wherein $R^{14}$ is hydrogen or $C_1$-$C_6$ alkyl, and wherein t and u are independently integers of from 1 to 3; and

viii) -Z-$Ar^2$, wherein Z is absent or is $CH_2$, O, $-OCH_2-$, or S, and wherein $Ar^2$ is aryl having 6, 10, or 14 nuclear ring carbon atoms or is heteroaryl having 5 or 6 nuclear ring atoms of which at least one nuclear ring atom is O, S, or N; and

$R^4$ is:

a) hydrogen;

b) $C_1$-$C_6$ alkyl;

c) a pharmaceutically acceptable cation; or

d) a prodrug ester group.

The term "$C_1$-$C_6$ alkyl" refers to straight or branched chain aliphatic hydrocarbon groups having from 1 to 6 carbon atoms, also referred to as lower alkyl. Examples of $C_1$-$C_6$ alkyl are methyl, ethyl, propyl, butyl, pentyl, hexyl, and the isomeric forms thereof.

The term "$C_1$-$C_4$ haloalkyl" refers to straight or branched chain alkyl groups having from 1 to 4 carbon atoms in which a hydrogen atom is replaced with a halogen atom. Examples of $C_1$-$C_4$ haloalkyl are halomethyl; 1- and 2-haloethyl; 1-, 2-, and 3-halopropyl; 1-, 2-, 3-, and 4-halobutyl; and the isomeric forms thereof. Examples of halo are fluoro, chloro, bromo, and iodo.

The term "$C_2$-$C_6$ alkenyl" refers to straight or branched chain hydrocarbon groups having from 2 to 6 carbon atoms and possessing one carbon-carbon double bond. Examples of $C_2$-$C_6$ alkenyl are vinyl; allyl; 2- or 3-butenyl; 2-, 3-, or 4-pentenyl; 2-, 3-, 4-, or 5-hexenyl; and isomeric forms thereof.

The term "$C_4$-$C_{11}$ (cycloalkyl)alkyl" refers to straight or branched chain alkyl groups bearing a cycloalkyl group such that the total number of carbon atoms ranges from 4 to 11. As used herein, the term "cycloalkyl" refers to saturated monocyclic hydrocarbon groups having from 3 to 7 carbon atoms in the ring. Examples of $C_4$-$C_{11}$ (cycloalkyl)alkyl are cyclopropylmethyl, 2-cyclopropylethyl, 3-cyclopropylpropyl, cyclobutylmethyl, 2-cyclobutylethyl, cyclopentylmethyl, 2-cyclopentylethyl, cyclohexylmethyl, 2-cyclohexylethyl, cycloheptylmethyl, 2-cycloheptylethyl, and the like, and the isomeric forms thereof.

The term "$C_7$-$C_{14}$ aralkyl" refers to straight or branched chain alkyl groups bearing a phenyl or naphthyl group such that the total number of carbon atoms ranges from 7 to 14. Examples of $C_7$-$C_{14}$ aralkyl are benzyl, phenethyl, phenylpropyl, (1-naphthyl)methyl, (1-naphthyl)ethyl, (2-naphthyl)methyl, (2-naphthyl)-ethyl, and the like, and isomeric forms thereof.

The term "aryl having 6, 10, or 14 nuclear ring carbon atoms" refers to aromatic hydrocarbon substituents having one ring or having two or three fused rings. Examples of such aryl groups are phenyl, 1-naphthyl, 2-naphthyl, and various groups derived from anthracene and phenanthrene.

The term "$C_1$-$C_6$ alkoxy" refers to straight or branched chain alkyl oxy groups having from 1 to 6 carbon atoms. Examples of $C_1$-$C_6$ alkoxy are methoxy, ethoxy, propoxy, butoxy, pentyloxy, hexyloxy, and the isomeric forms thereof.

The term"$C_2$-$C_6$ alkylene" refers to aliphatic hydrocarbon chains substituted at two different carbon atoms. Examples of $C_2$-$C_6$ alkenyl are $-(CH_2)_2-$, $-(CH_2)_3-$, $-(CH_2)_4-$, $-(CH_2)_5-$, $-(CH_2)_6-$, and $-(CH_2)_7-$, as well as the isomeric forms thereof. As used herein, $C_2$-$C_6$ alkylene chains are taken with nitrogen atoms to form 1-azacycloalkyl groups: 1-azacyclopropyl, 1-cryocyclobutyl, 1-azacyclopentyl, 1-azacyclohexyl, and 1-azacycloheptyl.

The term "heteroaryl" refers to aromatic ring systems having 5 or 6 nuclear ring atoms of which at least one nuclear ring atom is O, S, or N. Examples of heteroaryl are 1-, 2-, or 3-pyrrolyl; 1-, 2-, or 4-imidazolyl; 2- or 3-thienyl; 2- or 3-furanyl; 2-, 3- or 4-pyridinyl; 2- or 4-pyrimidinyl; s-triazinyl; 3-, 4-, or 5-isoxazolyl; 3-, 4-, or 5-isothiazolyl; and the like.

The term "pharmaceutically acceptable acid addition salt" refers to a salt prepared from a compound of Formula I in which at least one substitutent $R^2$ or $R^3$ contains a basic nitrogen atom. Said salts may be prepared by contacting such compounds of Formula I with an inorganic or organic acid whose anion is generally considered suitable for human consumption or by using other methods known in the art such as ion exchange. Examples of pharmaceutically acceptable acid addition salts include the acetate, adipate, alginate, aspartate, benzoate, benzenesulfonate, bisulfate, butyrate, citrate, camphorate, camphorsulfonate, cyclopentanepropionate, dodecylsulfate, enthanesulfonate, fumarate, glucoheptanoate, gluconate, glycerophosphate, heptanoate, hexanoate, hydrochloride, hydrobromide, hydroiodide, 2-hydroxyethanesulfonate, lactate, maleate, maleate, methanesulfonate, 2-naphthalenesulfonate, nicotinate, pamoate, pectinate, 3-phenylpropionate, phosphate, pivalate, propionate, succinate, sulfate, tartrate, thiocyanate, tosylate, and undecanoate salts.

The term "pharmaceutically acceptable cation" refers to a positively charged inorganic or organic ion that is generally considered suitable for human consumption. Examples of pharmaceutically acceptable cations are hydrogen, alkali metal (lithium, sodium, and potassium), magnesium (1/2 $Mg^{++}$), calcium (1/2 $Ca^{++}$), ammonium, alkylammonium, dialkylammonium, trialkylammonium, tetraalkylammonium, diethanolaminium, triethanolaminium, and guanidinium ions, and protonated forms of lysine, benzathine, procaine, and choline. Cations may be interchanged by methods known in the art, such as ion exchange. Where compounds of Formula I are prepared in the carboxylic acid form (that is, where $R^4$ is hydrogen ion), addition of a base form of the cation (such as a hydroxide or a free amine) will yield the appropriate cationic form.

The term "prodrug ester group" refers to any one of several ester-forming groups that are hydrolyzed under physiological conditions. Examples of prodrug ester groups include pivaloyloxymethyl, acetoxymethyl, phthalidyl, indanyl, and methoxymethyl, and other such groups known in the art, including those described in detail in the U.S. Pat. No. 4,479,947, which is incorporated herein by reference.

Examples of halogen are fluorine, chlorine, bromine, and iodine.

Compounds of Formula I in which $R^4$ is hydrogen and in which at least one substituent $R^2$ or $R^3$ contains a basic nitrogen atom may form internal salts, or zwitterious, in solid forms or in solution. It is understood that the structures represented by Formula I are for convenience only and that the scope of this invention encompasses such zwitterionic forms.

It is also understood that the compounds of Formula I may form hydrates or other solvates from the

solvents in which they are prepared or from which they are crystallized. These hydrates or other solvates may be used *per se* or they may be dehydrated or desolvated by heating (for example, at about 70° C to 100° C) *in vacuo*.

## DESCRIPTION OF THE INVENTION

The compounds of this invention may be prepared by methods illustrated in the following Schemes. Unless otherwise specified, the various substituents are defined as for Formula I, above. Scheme A illustrates the preferred method for preparing compounds of Formula VIII (that is, Formula I in which $R^1$ is hydrogen).

SCHEME A

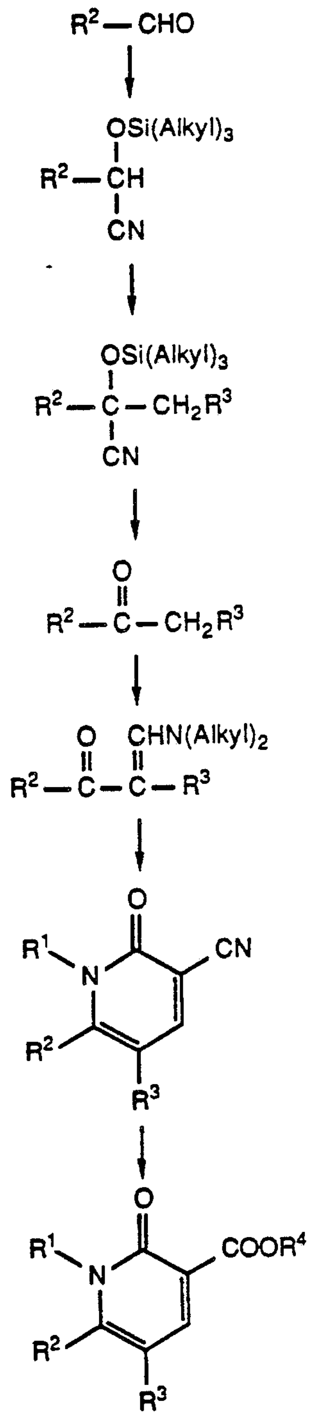

II

III

IV

V

VI

VII

VIII

An aldehyde of Formula II may be converted to the silyloxy nitrile of Formula III by reaction with a trialkylsilylcyanide, preferably trimethylsilylcyanide, in a suitable solvent and in the presence of a suitable catalyst. Suitable solvents are liquids, preferably organic liquids, in which the reactants may be dissolved or suspended but which are otherwise essentially chemically inert. Examples of suitable solvents include alkanes and cycloalkanes, ethers and cyclic ethers, such as diethyl ether and tetrahydrofuran; aromatic hydrocarbons, such as benzene and toluene; halocarbons, such as chloroform, dichloromethane, and ethylene chloride; and other solvents known in the art. A preferred solvent is dichloromethane. Suitable catalysts are substances, preferably inorganic salts, that facilitate the conversion of aldehydes of Formula II to silylated nitriles of Formula III but which are not permanently incorporated in the product compounds. A preferred catalyst is anhydrous zinc iodide.

Silyloxy nitriles of Formula III may be converted to compounds of Formula IV by any of several alkylation methods known in the art. A preferred alkylation method involves initial formation of a carbanion form of compound III by reaction with a suitable strong base in a suitable organic solvent. The carbanion is then allowed to react with a compound having the formula $R^3CH_2$-$X^1$, wherein $X^1$ is a suitable leaving group. Suitable strong bases are chemical substances capable of abstracting the active methane hydrogen adjacent to the nitrile group of compound III but which do not themselves form significant quantities of byproducts by reaction with other chemical reagents or reaction products. Examples of suitable strong bases include alkali metal alkoxides, such as sodium methoxide, sodium ethoxide, and potassium t-butoxide; alkali metal alkyls, such as n-butyllithium and t-butyllithium; metal hydrides, such as lithium hydride, sodium hydride, and potassium hydride; alkali metal amides, such as sodamide and lithium diisopropylamide; and other such bases known in the art. Suitable organic solvents are organic liquids in which the reactants may be dissolved or suspended but which are otherwise essentially chemically inert. Examples of suitable organic solvents include alkanes and cycloalkanes; ethers and cyclic ethers, such as diethyl ether, tetrahydrofuran, tetrahydropyran, dioxane, and the like; aromatic hydrocarbons, such as benzene and toluene; N,N-disubstituted amides, such as dimethylformamide and dimethylacetamide; N-substituted lactams, such as N-methylpyrrolidinone and N-methylpiperidinone; organonitriles, such as acetonitrile; and other solvents known in the art. Suitable leaving groups $X^1$ are groups sufficiently labile to permit the alkylation to occur. Examples of such groups $X^1$ are halogen, such as bromine, chlorine, and iodine; alkanesulfonate and arylsulfonate, such as methanesulfonate and p-toluenesulfonate; and other such leaving groups known in the art. A preferred leaving group $X^1$ is halogen, preferably chlorine or bromine.

Hydrolysis of silyloxy nitriles of Formula IV in a suitable acidic medium yields ketones of Formula V. Suitable acidic media for hydrolysis are comprised of suitable strong acids in a mixture of water and a suitable organic cosolvent. Suitable strong acids include mineral acids, such as hydrochloric, hydrobromic, sulfuric, and phosphoric acids, and the like, and highly dissociated organic acids, such as trifluoroacetic, methanesulfonic, and p-toluenesulfonic acids, and the like. For acids normally supplied as aqueous solutions, additional water need not be added to the hydrolysis medium. Suitable organic cosolvents are water miscible organic liquids and may include alkanols, such as methanol, ethanol, propanol, and the like; alkanones, such as acetone, methyl ethyl ketone, and the like; and other such solvents known in the art. A preferred acidic medium for hydrolysis includes aqueous hydrochloric or sulfuric acid with methanol or acetone.

Compounds of Formula V may be converted to enamines of Formula VI by reaction with an acetal of a dialkylformamide in a suitable organic solvent. A preferred dialkylformamide acetal is dimethylformamide dimethyl acetal. Suitable organic solvents are organic liquids, preferably polar organic liquids, in which the formation of enamines VI may occur. A preferred organic solvents is dimethylformamide. Although the reaction occurs at room temperature, the reaction is more conveniently performed at elevated temperatures, preferably between about 50° C and about 90° C.

Cyclization of enamines of Formula VI using methods known in the art yields pyridone nitriles of Formula VII. A preferred cyclization method uses cyanoacetamide in a suitable organic solvent in the presence of a suitable base. Suitable organic solvents are organic liquids in which the reactants may be dissolved or suspended and in which the cyclization reaction may readily occur but which are otherwise essentially chemically inert. Suitable organic solvents include N,N-disubstituted amides, such as dimethylformamide and dimethylacetamide; N-substituted lactams, such as N-methylpyrrolidinone and N-methylpiperidinone; and other solvents known in the art. Suitable bases are chemical substances sufficiently basic to induce cyclization but which do not themselves form significant quantities of byproducts by undesired reactions with other chemical reagents or reaction products. Suitable bases include alkali metal carbonates, such as lithium, sodium, and potassium carbonate; alkali metal alkoxides, such as sodium methoxide, sodium ethoxide, and potassium t-butoxide; and other bases known in the art. Preferred cyclization conditions employ sodium methoxide in dimethylformamide.

Solvolysis of nitriles of Formula VII using methods known in the art yields carboxylates of Formula VIII. In general, solvolysis may be performed using either acidic or basic conditions. For free carboxylic acids of Formula VII wherein $R^4$ is hydrogen, solvolysis may be performed in aqueous media. Suitable aqueous acidic media include mineral acids, such as hydrochloric, hydrobromic, and sulfuric acids, preferably at temperatures of about 50° C to 100° C; mixtures of mineral acids and alkanoic acids, preferably hydrobromic acid in acetic acid; and other such aqueous acidic media known in the art. Suitable aqueous basic media include alkali metal hydroxides, such as lithium, sodium, and potassium hydroxide, in water or in aqueous organic solutions, such as aqueous methanol or ethanol.

For carboxylate esters of Formula VIII wherein $R^4$ is $C_1$-$C_6$ alkyl, solvolysis may be performed in appropriate $C_1$-$C_6$ alkanols from which water is substantially excluded and to which suitable acids or bases

are added. Suitable acids include hydrogen halides, such as anhydrous hydrogen chloride and hydrogen bromide; concentrated sulfuric acid; highly dissociated organic acids, such as trifluoroacetic, methanesulfonic, and *p*-toluenesulfonic acids; and other such acids known in the art. Suitable bases include alkali metal alkoxides, preferably corresponding to the alkanol used as solvent, such as sodium methoxide, sodium ethoxide, and the like. Suitable bases may also be generated *in situ*, for example by adding an alkali metal such as sodium or potassium to the alkanol to be used for the solvolysis.

An alternative method for preparing carboxylate esters of Formula VII wherein $R^4$ is $C_1$-$C_6$ alkyl and a more general method for preparing compounds VIII wherein $R^4$ is a prodrug ester group involves esterification of corresponding carboxylic acids or salts thereof (that is, Formula VIII wherein $R^4$ is hydrogen or a cation). For example, esters in which $R^4$ is $C_1$-$C_4$ alkyl may be prepared by acid-catalyzed esterification using the corresponding $C_1$-$C_6$ alkanol as solvent; by forming an activated acyl compound, such as acid chloride, a carbonyl imidazolide, or a mixed anhydride, that is then allowed to react with the appropriate $C_1$-$C_6$ alkanol; or by using any other suitable esterification known in the art. Esters in which $R^4$ is a prodrug ester group may be conveniently prepared using the activated acyl method or other methods peculiar to a given prodrug ester.

Pharmaceutically acceptable salts of Formula VIII (that is, wherein $R^4$ is a pharmaceutically acceptable cation) may be prepared by any of several methods known in the art. The salts may be prepared from corresponding free acids (that is, wherein $R^4$ is hydrogen) by addition of a suitable base form of the cation or by ion exchange. Cation may further by exchanged using ion exchange methods. Suitable bases include alkali metal carbonates or hydroxides, such as lithium, sodium, or potassium carbonate or hydroxide; free amines; or hydroxide forms of amines, such as quaternary ammonium hydroxides. The preferred method depends upon the particular cation selected.

Scheme B illustrates a method for preparing compounds of Formula XI (that is, Formula I wherein $R^1$ is a substituent other than hydrogen) from either nitriles of Formula VII or carboxylates of Formula VIII.

SCHEME B

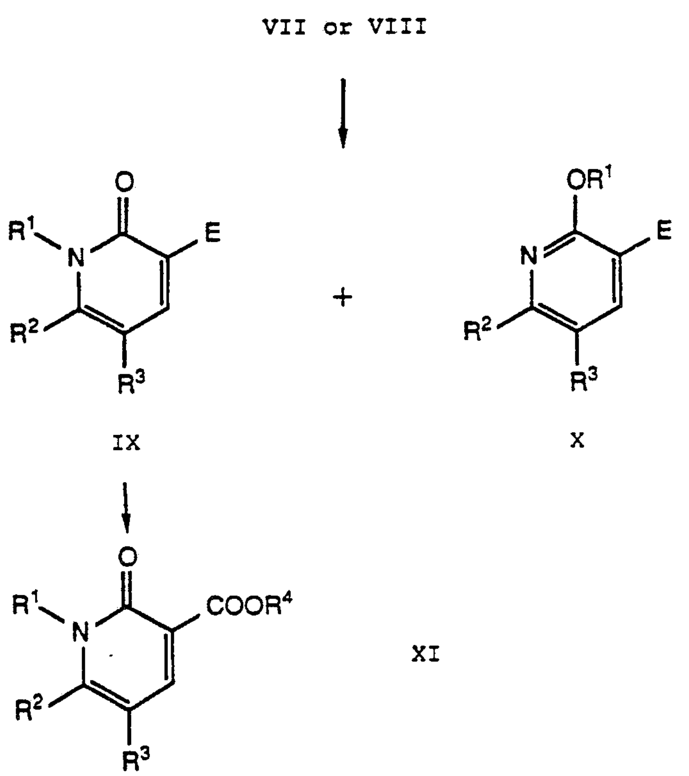

VII or VIII

IX

X

XI

Reaction of compounds VII or VIII with a compound having the formula $R^1$-$X^2$ (wherein $R^1$ is one of the groups defined above but other than hydrogen and wherein $X^2$ is a suitable leaving group) in a suitable

organic solvent and in the presence of a suitable base produces a mixture of N-substituted compounds of Formula IX and O-alkylated compounds of Formula X (wherein E represents the appropriate group CN or $COOR^4$). Suitable leaving groups $X^2$ are groups sufficiently labile to permit the reaction to proceed. Examples of such groups $X^2$ are halogen, such as bromine, chlorine, and iodine; alkanesulfonate or arylsulfonate, such as methanesulfonate and p-toluenesulfonate; and other such leaving groups known in the art. A preferred leaving group $X^2$ is a halogen. Where $R^1$ is $C_1$-$C_4$ haloalkyl, conditions may be selected so that only one halogen group reacts. Suitable organic solvents include N,N-disubstituted amides, such as dimethylformamide and dimethylacetamide; N-substituted lactams, such as N-methylpyrrolidinone and N-methylpiperidinone; halocarbons, such as chloroform and dichloromethane; dialkylsulfoxides, such as dimethylsulfoxide; organonitriles, such as acetonitrile; and other solvents known in the art. Preferred organic solvents are dimethylformamide and acetonitrile. Suitable bases are chemical substances sufficiently basic to promote the reaction and to prevent buildup of acidity but which do not themselves form significant quantities of byproducts by reacting with other chemical reagents or reaction products. Suitable bases include alkali metal carbonates, such as lithium, sodium, and potassium carbonate; other substantially dissociated metal carbonates, such as cesium carbonate; alkali metal alkoxides, such as sodium methoxide, sodium ethoxide, and potassium t-butoxide; alkali metal hydrides, such as sodium and potassium hydrides; and other such bases known in the art. Preferred bases include potassium carbonate, cesium carbonate, and sodium hydride.

The N-substituted carboxylates of Formula IX (that is, wherein E is $COOR^4$) may be separated from the corresponding O-substituted compounds X and, if desired, may subsequently be converted by methods discussed above to other compounds within the scope of Formula XI (that is, Formula I wherein $R^1$ is a substituent other than hydrogen).

The N-substituted nitriles of Formula IX (wherein E is CN) may also be separated from corresponding O-substituted compounds X and then converted to carboxylates of Formula XI using methods described above (see Scheme A) for converting nitriles of Formula VII to carboxylates of Formula VIII. The initially formed compounds XI may then be converted to other compounds within the scope of Formula XI (that is, Formula I wherein $R^1$ is a substituent other than hydrogen).

Certain functional groups within substituents $R^2$ and $R^3$ may be further manipulated. For example, thioether groups may be oxidized to corresponding sulfoxides and sulfones, preferably using a peroxycarboxylic acid such as m-chloroperoxybenzoic acid or p-nitroperoxybenzoic acid. Careful control of the oxidizing conditions permits preparation of sulfoxides without significant further oxidation to corresponding sulfones. Preferred conditions for preparing sulfoxides include using an approximately equimolar quantity of the peroxycarboxylic acid in a suitable organic solvent, such as chloroform or dichloromethane. Oxidation may be quenched, if desired, by adding aqueous sodium thiosulfate.

Sulfones may be prepared by either of two general methods. First, oxidation may be performed as described above except that at least a two-fold molar quantity of peroxycarboxylic acid is used. Alternatively, isolated sulfoxides may be further oxidized using oxidations essentially identical to those described for making sulfoxides from the thioethers.

Tertiary amino groups may similarly be converted to corresponding amine N-oxides.

Various aromatic ether groups may be converted to corresponding alcohols using methods known in the art. For example, ether groups may be cleaved under acidic conditions. Preferred conditions employ hydrobromic acid, either alone or in combination with acetic acid. Where the ether is a benzylic ether, the ether may alternatively be cleaved by hydrogenation over a catalyst such as palladium.

For the various oxidations and ether cleavages described above, compounds in which $R^4$ is hydrogen (or salts thereof) are preferably converted to corresponding esters, preferably methyl esters. Upon completion of the desired functional group changes, the ester group may be removed using solvolytic methods known in the art.

The preferred embodiments of this invention are methods and pharmaceutical compositions that employ compounds of the following general formula:

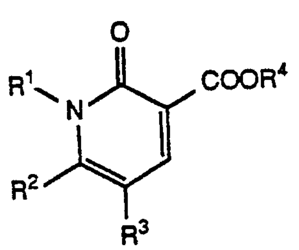

XII

wherein $R^1$ is defined as above for Formula I; wherein $R^2$ is phenyl substituted with one or more substituents defined as above for Formula I, or is (thienyl)thienyl; wherein $R^3$ is phenyl substituted with halogen, hydroxy, $C_1$-$C_6$ alkoxy, or benzyloxy, or is pyridinyl; and wherein $R^4$ is hydrogen or $C_1$-$C_6$ alkyl.

More preferred embodiments of this invention employ compounds of the following general formula:

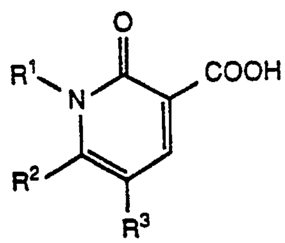

XIII

wherein $R^1$ is hydrogen, methyl, allyl, cyclopropylmethyl, or benzyl; wherein $R^2$ is phenyl substituted with one or more of fluorine, methoxy, methylthio, methylsulfinyl, methylsulfonyl, dimethylamino or the N-oxide thereof,

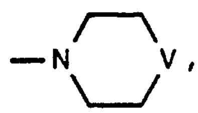

(wherein V is $CH_2$, O, or $NCH_3$), diethylaminoethoxy, or (2-pyridinyl)thio, or is 5-(2-thienyl)-2-thienyl; and wherein $R^3$ is phenyl substituted with fluorine, hydroxy, methoxy, or benzyloxy, or is 4-pyridinyl.

The compounds of the present invention exhibited antibacterial activity against various Gram-positive and Gram-negative bacteria. Representative pathogens that are sensitive to the antibacterial agents of this invention include various species of the following organisms: Staphylococcus, Streptococcus, Proteus, Escherichia, and Klebsiella. The antibacterial activities of the compounds illustrated in the Examples were tested by the following method.

Agar Diffusion Assay for Antibacterial Activity

A stock solution of each test compound was prepared at a concentration of 1.28 mg/ml using sterile distilled water. After initial solubilization, two-fold dilutions were prepared and added as one-ml portions to 15 x 100 mm petri plates. A nine-ml portion of trypticase soy agar (TSA) was melted and added to each plate. The test compound and agar were mixed and allowed to cool. Final concentrations of compounds was 128 µg/ml and lower, according to the dilutions used. Controls were prepared using norfloxacin, coumermycin A-1, or novobiocin, and solvent.

Each plate was inoculated using a multipoint inoculator with $10^5$ colony-forming units (cfu) of the appropriate bacterium. Each bacterium was stored at -70 °C before use and cultured in trypticase soy broth (TSB) at 35 °C for 18 hours. The cultures, containing ca. $10^9$ cfu/ml, were diluted as necessary with TSB to provide concentrations of $10^5$ cfu/inoculation spot. The test plates were then incubated at 35 °C for 20 hours. Minimum inhibitory concentrations (MIC) for each compound was the lowest concentration that allowed no growth, a barely visible haze, of five or fewer discrete colonies. Results are listed in Table VI (see Example 56).

The compounds and pharmaceutical compositions of the present invention are valuable antibacterial agents active against various Gram-positive and Gram-negative bacteria and accordingly find utility in human and veterinary medicine. The antibacterials of this invention are not limited to utility as medicaments; they may be used in all manner of industry, for example, as additives to animal feed, as preservatives of food, as disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in white water of paper mills to inhibit the growth of harmful bacteria.

Regardless of the route of administration selected, pharmaceutically effective amounts of the compounds of the present invention are formulated into pharmaceutically acceptable dosage forms by conventional methods known to those skilled in the art. The compounds may also be formulated using pharmaceutically acceptable acid or base addition salts where appropriate. Moreover, the compounds or their salts may be used in a suitable hydrated form. The compounds of this invention may be used in any of a variety of pharmaceutical preparations. The compounds may be administered in such oral dosage forms as tablets, capsules, pills, powders, granules, elixirs, or syrups. The compounds may also be administered intravascularly, intraperitoneally, subcutaneously, intramuscularly, or topically using forms known to the pharmaceutical art.

Compositions for injection, a preferred route of delivery, may be prepared in unit dosage form or in multidose containers. The compositions may take such forms as suspensions, solutions, or emulsions in oily or aqueous vehicles, and may contain formulatory agents. Alternatively, the active ingredient may be in powder form for reconstitution at the time of delivery with a suitable vehicle, such as sterile water. Topical applications may be formulated in hydrophobic or hydrophilic bases as ointments, creams, lotions, paints, or powders.

For the orally administered pharmaceutical compositions and methods of the present invention, the foregoing active ingredients will typically be administered in admixture with suitable pharmaceutical diluents, excipients, or carriers (collectively referred to herein as "carrier" materials) suitably selected with respect to the intended form of administration, that is, oral tablets, capsules, elixirs, syrups, and the like, and consistent with conventional pharmaceutical practices. For example, for oral administration in the form of tablets or capsules, the active drug components may be combined with any oral non-toxic pharmaceutically acceptable inert carrier such as lactose, starch, sucrose, cellulose, magnesium stearate, dicalcium phosphate, calcium sulfate, mannitol, and the like, or various combinations thereof; for oral administration in liquid form, the active drug components may be combined with any oral non-toxic pharmaceutically acceptable inert carrier such as water, saline, ethanol, polyethylene glycol, propylene glycol, corn oil, cottonseed oil, peanut oil, sesame oil, benzyl alcohol, various buffers, and the like, or various combinations thereof. Moreover, when desired or necessary, suitable binders, lubricants, disintegrating agents, and coloring agents can also be incorporated in the mixture. Suitable binders include starch, gelatine, natural sugars, corn sweeteners, natural and synthetic gums such as acacia, sodium alginate, carboxymethylcellulose, polyethylene glycol, and waxes, or combinations thereof. Lubricants for use in these dosage forms include boric acid, sodium benzoate, sodium acetate, sodium chloride, and the like, or combinations thereof. Disintegrators include without limitation, starch, methylcellulose, agar, bentonite, guar gum, and the like, or combinations thereof. Sweetening and flavoring agents and preservatives can also be included where appropriate.

The dosage to be administered depends to a large extent upon the condition and size of the subject being treated as well as the route and frequency of administration, the parenteral route by injection being preferred for generalized infections. Such matters, however, are left to the routine discretion of the therapist according to the principles of treatment well known in the antibacterial art. In general, a daily dosage consists of from about 5 to about 50 mg of active ingredient per kg of body weight of the subject in one or more treatments per day. A preferred daily dosage for adult humans lies in the range of from about 5 to 30 mg of active ingredient per kg of body weight. Another factor influencing the precise dosage regimen, apart from the nature of the infection and peculiar identity of the individual being treated, is the molecular weight of the chosen species of this invention.

The compositions for human delivery per unit dosage, whether liquid or solid, may contain from 0.1% to 99% of active material, the preferred range being from about 10-60%. The composition will generally contain from about 15 mg to about 1500 mg of the active ingredient; however, in general, it is preferable to employ a dosage amount in the range of from about 250 mg to 1000 mg. In parenteral administration, the unit dosage is usually the pure compound of Formula I in sterile water solution or in the form of a soluble powder intended for such a solution.

The preferred method of administration of the antibacterial agent of Formula I is parenteral by i.v. infusion, i.v. bolus, or i.m. injection.

For adults, 5-30 mg of Formula I antibacterial per kg of body weight given 2, 3, or 4 times per day is preferred. Preferred dosage is 250 mg to 1000 mg of the antibacterial agent of Formula I given two (b.i.d.), three (t.i.d.), or four (q.i.d.) times per day. More specifically, for mild infections, particularly of the urinary tract, a dose of 250 mg t.i.d. or q.i.d. is recommended. For moderate infections against highly susceptible gram positive and gram negative organisms, a dose of 500 mg t.i.d. or q.i.d. is recommended. For severe, life-threatening infections against organisms at the upper limits of sensitivity to the antibiotic, a dose of 1000 mg t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

The following examples further illustrate details for the preparation of the compounds of this invention. The invention, which is set forth in the foregoing disclosure, is not to be construed or limited either in spirit or in scope by these examples. Those skilled in the art will readily understand that known variations in the conditions and processes of the following preparative procedures can be used to prepare these compounds. All temperatures are degrees Celsius unless otherwise noted.

DESCRIPTION OF THE PREFERRED EMBODIMENTS

EXAMPLE 1

1-(4-Dimethylaminophenyl)-2-(4-fluorophenyl)-1-ethanone

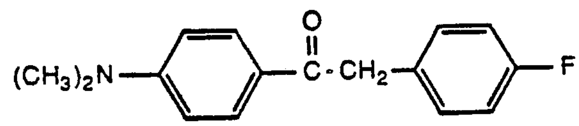

To a solution of p-dimethylaminobenzaldehyde (1.49 g, 10 mmole) and anhydrous zinc iodide (ca. 100 mg) in dichloromethane (20 ml) is added trimethylsilylcyanide (1.40 ml, 10.5 mmole). The reaction is stirred under an inert atmosphere at room temperature for 20 hours. The reaction is then concentrated and the residue taken up in anhydrous diethyl ether, slurried with anhydrous magnesium sulfate, and filtered. Concentration of the filtrate affords α-trimethylsilyloxy-(4-dimethylaminophenyl)acetonitrile (2.46 g), m.p. 57-59°.

A solution of the nitrile (10 mmole) in tetrahydrofuran (10 ml) is added via cannula to a cold (-60°) solution of lithium diisopropylamide (10 mmole) in tetrahydrofuran (10 ml). The resulting suspension is stirred in the cold for 30 minutes. A solution of p-fluorobenzyl bromide (2.0 g, 10.5 mmole) in tetrahydrofuran (5 ml) is then added via cannula. The cooling bath is removed and the reaction mixture stirred for two hours. A saturated aqueous solution of ammonium chloride (25 ml) is then added, followed by diethyl ether (50 ml). The organic phase is separated and washed with ammonium chloride solution, dried over sodium sulfate, filtered, and concentrated to afford 2-(4-dimethylaminophenyl)-3-(4-fluorophenyl)-2-trimethylsiloxypropionitrile.

The crude nitrile is taken up in methanol (15 ml) and 1M sulfuric acid (20 ml) and stirred at room temperature for 16 hours. The pH is adjusted to 7.5 with 2M sodium hydroxide, followed by extraction with dichloromethane. After drying and concentration of the extracts, the title compound is isolated as a light yellow solid (1.57 g), m.p. 149-150°. Structure assignment is confirmed by nmr spectroscopy.
nmr ($CDCl_3$): δ (ppm) 3.05 (s); 4.16 (s); 6.65 (d); 6.97 (t); 7.22 (dd); 7.91 (d)

EXAMPLES 2-14

1-(Aryl and heteroaryl)-2-(aryl and heteroaryl)-1-ethanones

The compounds listed in Table I are prepared by the method described in Example 1 using appropriate aldehydes and arylmethyl or heteroarylmethyl halides. Melting points (°C) are listed to the right of each structure. Nuclear magnetic resonance (nmr) data (obtained in $CDCl_3$) are presented below the appropriate structure.

Where the 2-trimethylsilyloxypropionitrile derivative intermediates are insoluble in the methanol-sulfuric

acid mixture, the following alternative method is used: The crude 2-trimethylsilyloxypropionitrile derivative (10 mmole) is dissolved in acetone (100 ml). Sulfuric acid (1M, 12.5 ml) or hydrochloric acid (12M, 1 ml) is added and the reaction is stirred 16-20 hours at room temperature. The acetone is removed in vacuo, water is added, and the pH is adjusted to 7-8. The product ketone is then isolated as described in Example 1.

TABLE I

| Example | Compound | m.p. (°C) |
| --- | --- | --- |
| 2 | 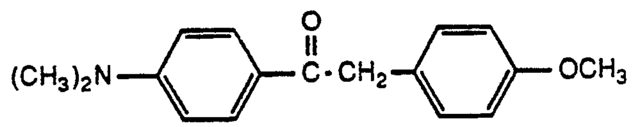 | 130-132° |
| δ (ppm) | 3.04(s); 3.78(s); 4.12(s); 6.64(d); 6.84(d); 7.2(d); 7.92(d) | |
| 3 | 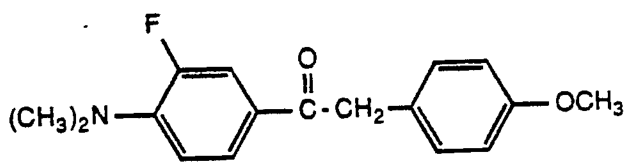 | 58-62° |
| δ (ppm) | 3.00(s); 3.02(s); 3.08(s); 3.09(s); 3.90(s); 4.14(s); 6.66-6.92(m); 7.2(d);7.58-7.78(m) | |
| 4 | 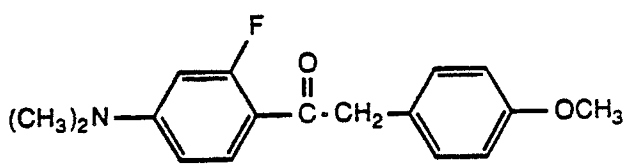 | 106-108° |
| δ (ppm) | 3.02(s); 3.79(s); 4.15(d); 6.25(dd); 6.47(dd); 6.85(d); 7.2(d); 7.83(t) | |

5 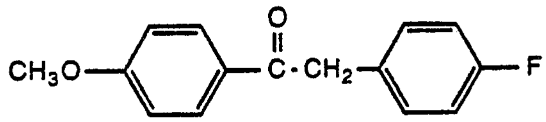 102-104°

δ (ppm) 3.90(d); 4.24(s); 6.92-7.1(m); 7.22(m); 8.0(d)

6 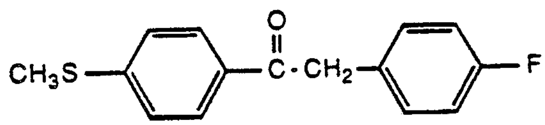 132-134°

δ (ppm) 2.56(s); 4.25(s); 7.03(t); 7.2-7.33(m); 7.93(d)

7 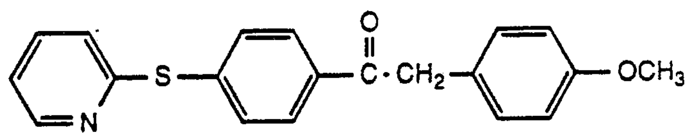 101-103°

δ (ppm) 3.80(s); 4.23(s); 6.88(d); 7.1-7.24(m); 7.58(d); 7.98(d); 8.5(br d)

8 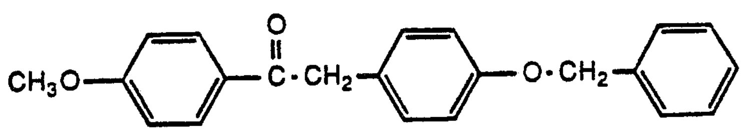 118-120°

δ (ppm) 3.86(s); 4.20(s); 5.06(s); 6.94(d); 7.20(d); 7.3-7.5(m); 8.0(d)

9 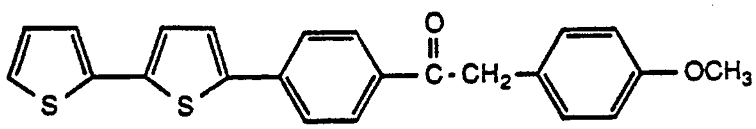 129-131°

δ (ppm) 3.80(s); 4.11(s); 6.87(d); 7.05(dd); 7.16(d); 7.2-7.35(m); 7.65(d)

10 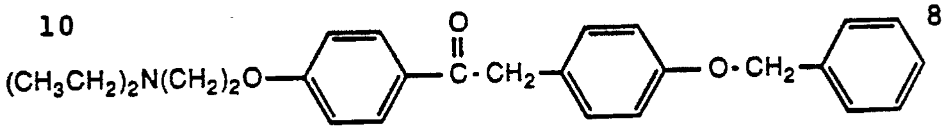 80-81°

δ (ppm) 1.1(t); 2.68(q); 2.92(t); 4.14(t); 4.20(s); 5.07(s); 6.96(d); 7.2(d); 7.32-7.5(m); 8.0(d)

11 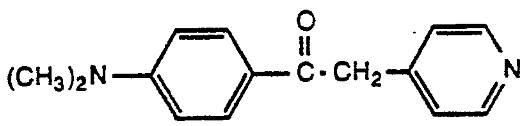 188-191°

δ (ppm) 3.08(s); 4.2(s); 6.66(d); 7.24(d); 7.90(d); 8.52(d)

12 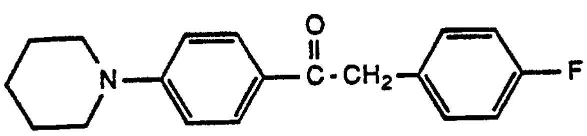

δ (ppm) 2.40(s); 2.6(t); 3.44(t); 4.20(s); 6.90(d); 6.96-7.1(m); 7.2-7.3(m); 7.95(d)

**13**

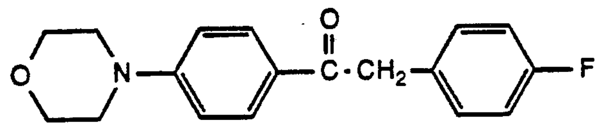

δ (ppm) 3.44(t); 3.88(t); 4.22(s); 6.9(d); 6.98-7.2(m); 7.2-7.3(m); 7.96(d)

14

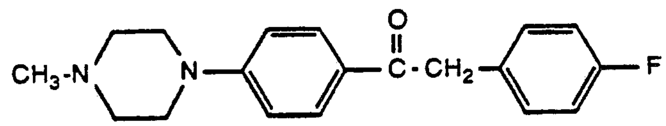

δ (ppm) 2.40(s); 2.6(t); 3.44(t); 4.20(s); 6.90(d); 6.96-7.1(m); 7.2-7.3(m); 7.95(d)

## EXAMPLE 15

6-[4-(Dimethylamino)phenyl]-1,2-dihydro-5-(4-methoxyphenyl)-2-oxo-3-pyridinenitrile

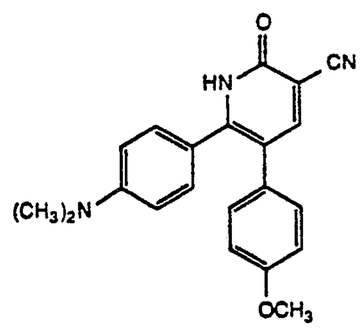

A solution of 1-(4-dimethylaminophenyl)-2-(4-methoxyphenyl)-1-ethanone (1.0 g, 3.7 mmole; see Example 2) and N,N-dimethylformamide dimethyl acetal (2.0 ml, 14.8 mmole) in dimethylformamide (20 ml) is heated at 75° for 20 hours. The reaction mixture is then concentrated to an oil that is dissolved in dimethylformamide (20 ml). To the resulting solution is added cyanoacetamide (0.345 g, 4.1 mmole) and methanol (0.34 ml, 8.2 mmole). This solution is added dropwise to a slurry of sodium hydride (0.20 g, 8.3 mmole) in dimethylformamide (5 ml). After the addition is completed, the reaction mixture is heated at 95° for four hours. After cooling to room temperature, the reaction mixture is poured into 0.5M sodium dihydrogen phosphate solution (125 ml). The solid is recovered by filtration and dried, then washed with diethyl ether to give the title compound (0.905 g), m.p. 273-276°. Structure assignment is confirmed by nmr spectroscopy.
nmr ($CDCl_3$): δ (ppm) 3.0(s); 3.82(s); 6.59(d); 6.82(d); 7.03(d); 7.12(d); 8.84(s)

## EXAMPLES 16-28

5-(Aryl and heteroaryl)-6-(aryl and heteroaryl)-1,2-dihydro-2-oxo-3-pyridinenitriles

The compounds listed in Table II are prepared by the method described in Example 15 using appropriate ketone intermediates. Melting points (°C) are listed to the right of each structure. Nuclear magnetic resonance (nmr) data (obtained in $CDCl_3$) are presented below the appropriate structure.

TABLE II

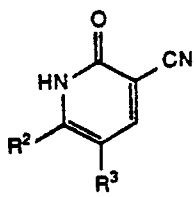

| Example | $R^2$ | $R^3$ | m.p. (°C) |
|---|---|---|---|
| 16 | 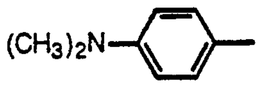 | 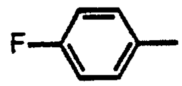 | 278-281° |
| δ (ppm) | 3.0(s); 6.62(d); 6.95-7.2(m); 7.94(s) | | |
| 17 | 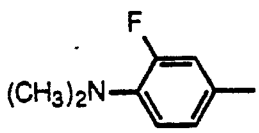 | 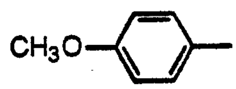 | 264-266° |
| δ (ppm) | 2.94(pair s); 3.8(s); 6.7-7.1(m); 7.97(s) | | |
| 18 | 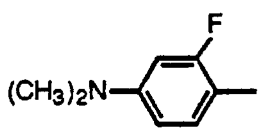 | 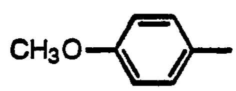 | 262-264° |
| δ (ppm) | 3.0(s); 3.84(s); 6.22-6.42(pair dd) 6.82(d); 6.90-7.1(m + d); 7.92(s) | | |

19 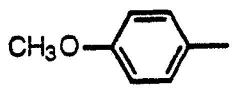 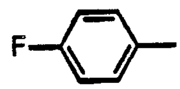 272-274°

δ (ppm) 3.90(s); 6.92(d); 7.04(d); 7.05-7.12(m); 7.26(d); 7.96(s)

20 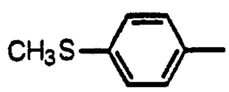 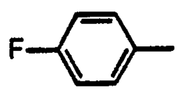 271-272°

δ (ppm) 2.50(s); 6.95-7.14(m); 7.22(s); 7.92(s)

21 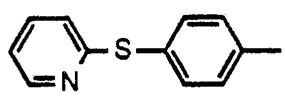 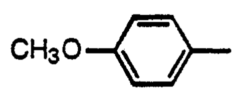 240-243°

δ (ppm) 3.82(s); 6.82(d); 6.98(d) 7.08-7.2(m); 7.32(d); 7.51(d); 7.64(t); 7.95(s); 8.47(d)

22 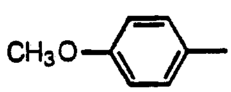 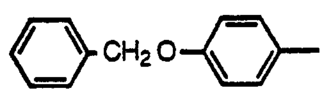 223-226°

δ (ppm) 3.84(s); 5.07(s); 6.92-7.05(m); 7.26(d); 7.32-7.5(m); 7.92(s)

23 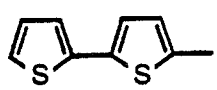 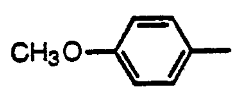 251-253°

δ (ppm) 3.88(s); 6.92-7.30(m); 7.73(d); 7.81(s)

24

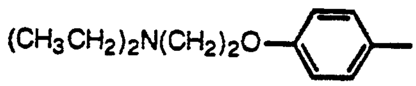 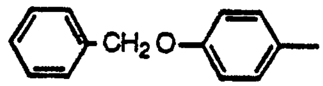

δ (ppm) 1.18(t); 2.80(br t); 3.03(br t); 4.18(br t); 5.06(s); 6.8-7.05(m); 7.24(d); 7.3-7.5(m); 7.91(s)

25

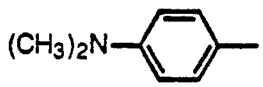 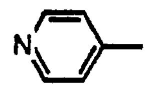

275-277° (dec.)

δ (ppm) 3.03(s); 6.6(d); 7.1(br); 7.15(d); 7.88(s); 8.55(br)

26

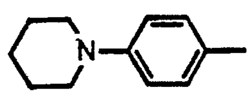 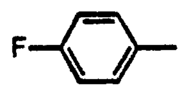

δ (ppm) 1.66(br s); 2.30(br s); 6.80(d); 6.9-7.2(m); 7.85(s)

27

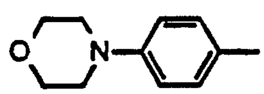 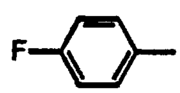

δ (ppm) 3.3(br t); 3.89(br t); 6.84(d); 6.96-7.18(m); 7.24(d); 8.92(s)

28

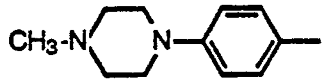 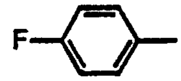

149-152°

δ (ppm) 2.36(s); 2.60(br t); 3.36(br t); 6.80(d); 6.92-7.12(m); 7.18(d); 7.88(s)

EXAMPLE 29

6-[4-(Dimethylamino)phenyl]-1,2-dihydro-5-(4-methoxyphenyl)-2-oxo-3-pyridinecarboxylic acid

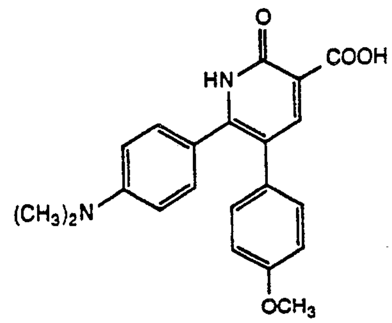

The title nitrile of Example 15 (3.0 g) is heated at 120° in 6M sulfuric acid (30 ml) for 17 hours. After cooling to room temperature, the reaction is poured into ice water (300 ml) and the pH is adjusted to ca. 5. The solid is collected by filtration and briefly air dried. It is then washed with diethyl ether-methanol (9:1), followed by acetonitrile. The solid is air dried to give the pure title compound (2.65 g), m.p. 273-277° (dec.). Structure assignment is confirmed by nmr spectroscopy.
nmr (($(CD_3)_2SO$): δ (ppm) 2.95(s); 3.75(s); 6.61(d); 6.86(d); 7.1(d); 7.16(d); 8.15(s)

EXAMPLE 30

6-[4-Dimethylamino)phenyl]-1,2-dihydro-5-(4-hydroxyphenyl)-2-oxo-3-pyridinecarboxylic acid, Method A

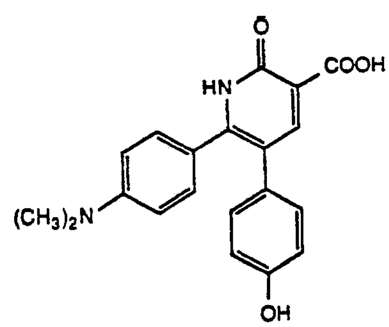

The title carboxylic acid of Example 29 (2.6 g) is dissolved in 48% hydrobromic acid (100 ml). The solution is purged with nitrogen for 15 minutes and then heated under nitrogen at 90° for 18 hours. After cooling to room temperature the reaction is added to 50% sodium hydroxide (47 ml) and ice (300 g). The resulting solution is treated with charcoal and filtered, and the pH of the filtrate is adjusted to 4 with acetic acid. The precipitated product is filtered, washed sequentially with water and diethyl ether-methanol (9:1), and dried to afford the title compound (2.47 g), m.p. >250°. Structure assignment is confirmed by nmr spectroscopy.
nmr ($(CD_3)_2SO$): δ (ppm) 2.96(s); 6.64(d); 6.70(d); 6.96(d); 7.14(d); 8.17(s); 9.50(s)

EXAMPLE 31

6-[4-Dimethylamino)phenyl]-1,2-dihydro-5-(4-hydroxyphenyl)-2-oxo-3-pyridinecarboxylic acid, Method B

The title nitrile of Example 15 (1.08 g) is heated in a mixture of 48% hydrobromic acid (10 ml) and 30% hydrogen bromide in acetic acid (10 ml) at 110° for 17 hours. After cooling, the reaction mixture is added to 50% sodium hydroxide (12 ml) and ice (50 g). The homogeneous solution that is obtained after the further addition of sodium hydroxide is treated with charcoal and filtered. The pH of the filtrate is adjusted to 5, and the solids are collected by filtration and washed with water. The solids are washed further with acetonitrile containing a small quantity methanol and then with pure acetonitrile. The title compound is obtained as a solid 0.964 g).

EXAMPLE 32

5-(4-Fluorophenyl)-1,2-dihydro-6-(4-methoxyphenyl)-2-oxo-3-pyridinecarboxylic acid

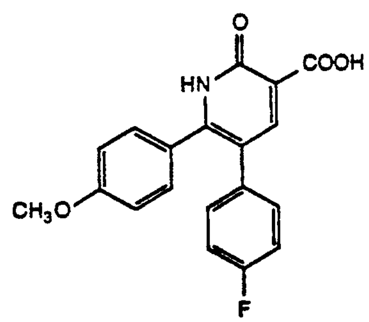

A solution of 5-(4-fluorophenyl)-1,2-dihydro-6-(4-methoxyphenyl)-2-oxo-3-pyridinenitrile (0.250 g; see Example 19) in a mixture of ethanol (5 ml), water (5 ml), and 50% sodium hydroxide (3 ml) is heated at 90° for 48 hours, after which time evolution of ammonia ceases. After cooling, the reaction solution is added to a mixture of water (50 ml) and 12M hydrochloric acid (7 ml). The resulting precipitate is stirred vigorously for 45 minutes at 50°. The solid is collected by filtration, washed several times with water, and dried to yield the title compound (0.220 g), m.p. 275-277° (dec.). Structure assignment is confirmed by nmr spectroscopy.
nmr ($CDCl_3$): 3.88(s); 6.90(d); 7.02(d); 7.06-7.2(m); 7.14(d); 8.6(s)

EXAMPLES 33-44

5-(Aryl and heteroaryl)-6-(aryl and heteroaryl)-1,2-dihydro-2-oxo-3-pyridinecarboxylic acids

The compounds listed in Table III are prepared by the methods described in Examples 29-32 using appropriate nitrile intermediates. Melting points (°C) are listed to the right of each structure. Nuclear magnetic resonance (nmr) data (obtained in $CDCl_3$ unless otherwise noted) are presented below the appropriate structure.

## TABLE III

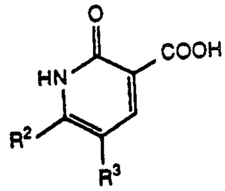

| Example | $\underline{R}^2$ | $\underline{R}^3$ | m.p. (°C) |
| --- | --- | --- | --- |
| 33 | 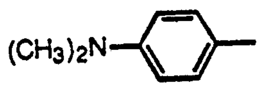 | 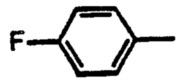 | 267–270° (dec.) |

δ (ppm) 3.04(s); 6.64(d); 7.01(t); 7.1-7.24(m); 8.53(s)

34 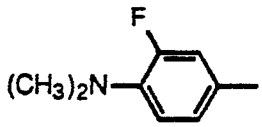 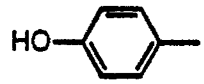 280-283° (dec.)

δ (ppm) $((CD_3)_2SO)$ 2.85(s); 6.70(d); 6.77-7.2(m); 7.28(br s); 8.2(s); 9.55(s)

35 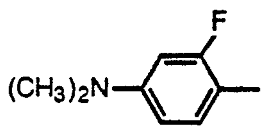 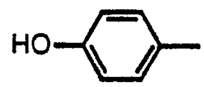 280-282° (dec.)

δ (ppm) 3.04(s); 3.06(s); 6.35(d); 6.7-7.2(m); 8.58(s)

36 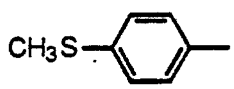 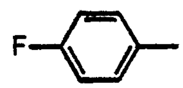 >350°

δ (ppm) 2.5(s); 6.9-7.2(m); 8.6(s)

37 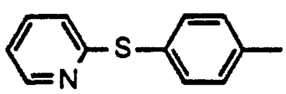 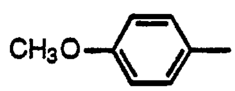 282-284° (dec.)

δ (ppm) $((CD_3)_2SO)$ 6.68(d); 6.94(d); 7.0(d); 7.24(br t); 7.38(d); 7.56(d); 7.72(br t); 8.3(s); 8.45(br d); 9.52(br s)

38 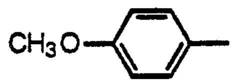 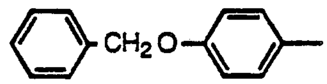 >350°

δ (ppm) 3.88(s); 5.1(s); 6.73(br d); 7.08(br d); 7.2-7.5(m); 8.74(s)

39 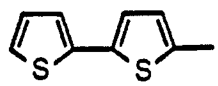 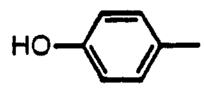 280-282°

δ (ppm) ($(CD_3)_2SO$) 6.8(d); 7.02-7.16(m); 7.18-7.4(m); 7.5-7.6(m); 7.7(br); 8.02(s); 8.15(s); 9.62(s); 9.66(s)

40 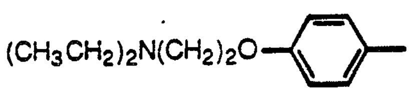 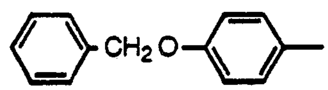 83-88°

δ (ppm) 1.44(t); 3.28(br); 3.46(br); 4.62(br); 5.04(s); 6.9(br d); 7.02(d); 7.2-7.5(m); 8.58(s)

41 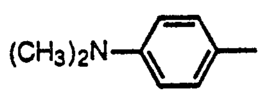 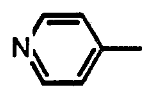 289-290° (dec.)

δ (ppm) 3.05(s); 6.63(d); 7.1-7.25(m); 8.57(s + br s)

42 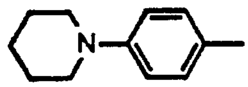 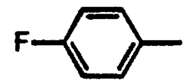 285-290° (dec.)

δ (ppm) 1.68(br s); 3.33 (br m); 6.94(d); 7.02(t); 7.1-7.25(m); 8.56(s)

43 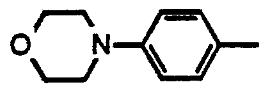 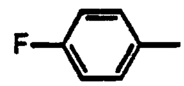 288-291° (dec.)

δ (ppm) 3.3(t); 3.88(t); 6.84(d); 7.01(t); 7.1-7.3(m); 8.58(s)

44 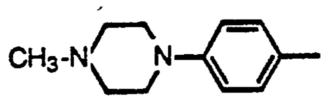 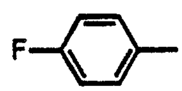 171-174°

δ (ppm) 2.44(s); 2.68(br s); 3.38(br s); 6.81(d); 7.0(t); 7.1-7.3(m); 8.52(s)

## EXAMPLE 45

1,2-Dihydro-5-(4-hydroxyphenyl)-6-(4-methoxyphenyl)-2-oxo-3-pyridinecarboxylic acid

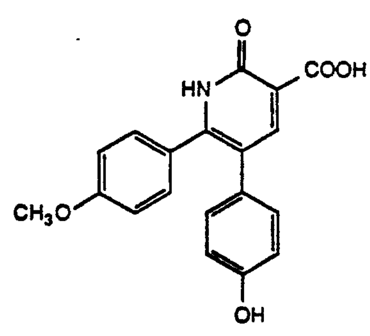

1,2-Dihydro-6-(4-methoxyphenyl)-2-oxo-5-[4-(phenylmethoxy)phenyl]-3-pyridinecarboxylic acid (2.35 g; see Example 38) is added to methanol previously saturated with hydrogen chloride at 0°. The mixture is brought to 60° and aged for eight hours, then cooled to room temperature and stored for 20 hours. The reaction solution is concentrated under vacuum. The residue is treated with water, adjusted to pH 6.5 with sodium hydroxide solution, and extracted with dichloromethane. The extracts are dried and concentrated to afford the methyl ester of title compound (0.930 g).

Hydrolysis of the methyl ester in dilute aqueous sodium hydroxide containing methanol and acidification of the resulting solution affords the title compound, m.p. 270-272°. Structure assignment is confirmed by nmr spectroscopy.

nmr (($(CD_3)_2SO$): δ (ppm) 3.78(s); 6.66(d); 6.91(pair d); 7.24(d); 8.22(s); 9.5(s)

## EXAMPLE 46

6-[4-(2-Diethylamino)ethoxy]phenyl-1,2-dihydro-5-(4-hydroxyphenyl)-2-oxo-3-pyridinecarboxylic acid

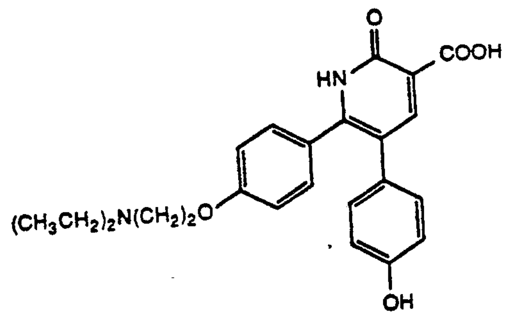

6-[4-[2-(Diethylamino)ethoxy]phenyl]-1,2-dihydro-2-oxo-5-[4-(phenylmethoxy)phenyl]-3-pyridinecarboxylic acid (see Example 40) is esterified using methanolic hydrogen chloride as described in Example 45. The crude product that is isolated contains a mixture of methyl esters in which the benzyloxy group is partially cleaved. A portion of this mixture (0.175 g) is dissolved in a mixture of methanol (3 ml) and 2N hydrochloric acid (0.2 ml). The reaction solution is hydrogenated over 10% palladium-charcoal (0.05 g) at 40 psi and room temperature for 2.5 hours. The reaction mixture is filtered and concentrated to a glass. Trituration with diethyl ether and filtration yields the methyl ester of the title compound as the hydrochloride salt.

Hydrolysis of the methyl ester in dilute aqueous sodium hydroxide containing methanol and acidification of the resulting solution affords the title compound, m.p. 182-185°. Structure assignment is confirmed by nmr spectroscopy.

mr ($CD_3OD$): δ (ppm) 1.3(br t); 2.9-3.5(br m); 4.3(br); 6.65(d); 6.8-7.0(br m); 8.22(s)

## EXAMPLE 47

5-(4-Fluorophenyl)-1,2-dihydro-6-[4-(methylsulfonyl)phenyl]-2-oxo-3-pyridinecarboxylic acid

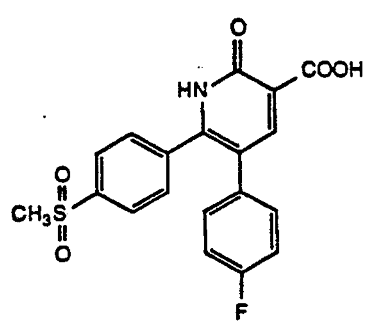

A solution of 5-(4-fluorophenyl)-1,2-dihydro-6-[4-(methylthio)phenyl]-2-oxo-3-pyridine carboxylic acid (1.0 g; see Example 36) in methanol (50 ml) is saturated with hydrogen chloride and kept at 50° for 18 hours, then concentrated under vacuum. The residue is taken up in water, adjusted to pH 6, and extracted with dichloromethane. Concentration of the organic extracts affords the ester derivative methyl 5-(4-fluorophenyl)-1,2-dihydro-6-[4-(methylthio)phenyl)-2-oxo-3-pyridinecarboxylate.

A solution of the ester derivative (0.074 g) in chloroform (2.0 ml) is treated with a 2.5-fold molar excess of purified 3-chloroperoxybenzoic acid (0.086 g). After one hour at room temperature, the reaction solution is washed sequentially with 10% sodium bicarbonate solution and water, dried over sodium sulfate, filtered, and concentrated to afford the methyl ester of the title compound (0.072 g).

Hydrolysis of the methyl ester in dilute aqueous sodium hydroxide containing methanol and acidification of the resulting solution affords the title compound, m.p. 320-321 ° (dec.). Structure assignment is confirmed by nmr spectroscopy.

nmr ($CDCl_3$ + $CD_3OD$): δ (ppm) 3.12(s); 6.96-7.10(m); 7.3-7.45(m); 7.5(d); 7.92-8.05(d + m); 8.64(s)

## EXAMPLES 48-50

### Sulfinyl and N-oxide derivatives

The compounds listed in Table IV are prepared by the method described in Example 47 except that one molar equivalent of 3-chloroperoxybenzoic acid is used for each mole of ester oxidized. Melting points (° C) are listed to the right of each structure. Nuclear magnetic resonance (nmr) data (obtained in $CDCl_3$ unless otherwise noted) are presented below the appropriate structure.

TABLE IV

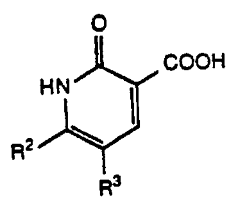

| Example | $R^2$ | $R^3$ | m.p. (°C) |
|---|---|---|---|
| 48 | 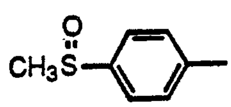 | 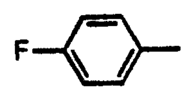 | 292-293° (dec.) |

δ (ppm) 2.79(s); 6.90-7.15(m); 7.48(d); 7.70(d); 8.66(s)

| Example | $R^2$ | $R^3$ | m.p. (°C) |
|---|---|---|---|
| 49 | 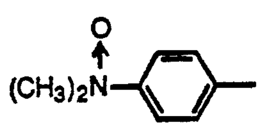 | 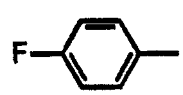 | 305-310° (dec.) |

δ (ppm) ($CD_3OD$) 3.60(s); 7.02(br m); 7.16(br); 7.5(br); 7.96(br d)

50 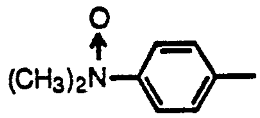 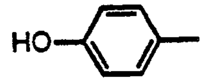 >350°

δ (ppm) ($CD_3OD$) 3.56(s); 6.70(m); 6.90(m); 7.48(br); 7.92(br); 8.3(br)

EXAMPLE 51

6-[4-(Dimethylamino)phenyl]-1,2-dihydro-5-(4-hydroxyphenyl)-1-methyl-2-oxo-3-pyridinecarboxylic acid

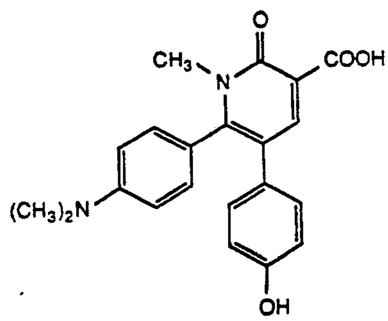

A solution of 6-[4-(dimethylamino)phenyl]-1,2-dihydro-5-(4-methoxyphenyl)-2-oxo-3-pyridinenitrile (0.690 g, 2.0 mmole; see Example 15) in dimethylformamide (14 ml) is treated with cesium carbonate (0.715 g, 2.2 mole) and methyl iodide (140 µl, 2.2 mmole). After stirring for 1 hour at room temperature, the reaction mixture is concentrated and the residue is partitioned between 1 M sodium dihydrogen phosphate solution and chloroform. The organic layer is dried and concentrated to a solid. This solid is boiled with diethylether, filtered, and washed with diethyl ether to afford 6-[4-(dimethylamino)phenyl]-1,2-dihydro-5-(4-methoxypehnyl)-1-methyl-2-oxo-3-pyridinenitrile as a solid (0.505 g).

The title compound (0.148 g), m.p. 277-281° (dec.), is then prepared from the nitrile using the methods described in Examples 29 and 30. Structure assignment is confirmed by nmr spectroscopy.

nmr ($CDCl_3$): δ (ppm) 3.03(s); 3.58(s); 6.5-6.9(m); 6.98(d); 8.52(s)

EXAMPLES 52-55

1-Substituted 6-[4-(Dimethylamino)phenyl]-5-(4-fluorophenyl)-1,2-dihydro-2-oxo-3-pyridinecarboxylic acids

The compounds listed in Table V are prepared by the method described in Example 51 (except for the hydrolysis step of Example 6) using 6-[4-(dimethylamino)phenyl]-5-(4-fluorophenyl)-1,2-dihydro-2-oxo-3-pyridinenitrile, (see Example 16). Melting points (°C) are listed to the right of each structure. Nuclear magnetic resonance (nmr) data (obtained in $CDCl_3$) are presented below the appropriate structure.

TABLE V

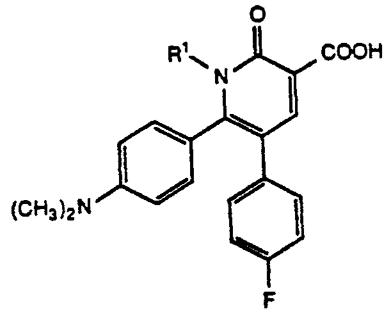

| Example | $R^1$ | m.p. (°C) |
|---|---|---|
| 52 | $CH_3$- | |
| δ (ppm) | 3.44(s); 2.98(s); 6.58(d); 6.7-7.2(m); 8.52(s) | |
| 53 | $CH_2$=CH-$CH_2$- | 175-177° |
| δ (ppm) | 2.98(s); 3.66(d); 4.96(d); 5.22(d); 5.9(m); 6.58(d); 6.9-7.2(m); 8.54(s) | |
| 54 | 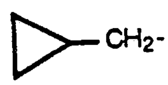 | 187-192° |
| δ (ppm) | 0.2(br); 0.4(br d); 1.0-1.8(br m); 2.98(s); 4.1(d); 6.6(d); 6.75-7.0(m); 8.50(s) | |
| 55 | 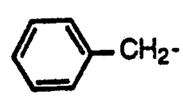 | 193-195° |
| δ (ppm) | 2.95(s); 5.36(s); 6.5(d); 6.72(d); 6.82-7.0(m); 7.25(obscured m); 8.58(s) | |

EXAMPLE 56

Antibacterial activity of representative 5-(aryl and heteroaryl)-6-(aryl and heteroaryl)-1,2-dihydro-2-oxo-3-pyridinecarboxylic acids

Table VI includes biological test results for representative compounds of this invention using the agar diffusion assay described above. The particular bacterial strains used are representative of organisms for which the compounds are useful as antibacterial agents.

| Organism[a] | Strain (MB No.) | 30 | 32 | 33 | 34 | 35 | 36 | 37 | 42 | 43 | 44 | 47 | 48 | 49 | 50 | 51 | 52 |
|---|---|---|---|---|---|---|---|---|---|---|---|---|---|---|---|---|---|
| | | Compounds (Ex. No.)/Minimum inhibitory conc. (μg/ml) | | | | | | | | | | | | | | | |
| St. aur. | 2865 | 0.25 | 2.0 | 2.0 | 2.0 | 0.5 | 8.0 | >128 | 1.0 | 4.0 | 32 | >128 | >128 | 1.0 | 4.0 | 1.0 | 2.0 |
| St. aur. | 4310 | 0.25 | 1.0 | 2.0 | 8.0 | 1.0 | 8.0 | >128 | 1.0 | 4.0 | 16 | >128 | >128 | 0.5 | 8.0 | 0.5 | 2.0 |
| Str. fae. | 2864 | 0.50 | 16.0 | 128 | 16 | 4.0 | 64 | >128 | 64 | 64 | 128 | >128 | >128 | 16 | 16 | 128 | >128 |
| Pr. mir. | 2830 | 8.0 | 32 | 128 | >128 | 32 | >128 | >128 | >128 | 128 | >128 | >128 | >128 | 64 | 128 | 32 | >128 |
| E. coli | 2891 | 0.25 | 16 | 16 | 4 | 1.0 | 64 | >128 | >128 | 16 | 8.0 | >128 | >128 | 32 | 8.0 | 0.5 | 2.0 |
| E. coli | 5157 | 0.25 | 4 | 8 | 16 | 1.0 | 16 | >128 | >128 | 8 | 16 | >128 | >128 | 4 | 8.0 | 2.0 | 4.0 |
| E. coli | 5158[b] | >128 | >128 | >128 | >128 | >128 | >128 | >128 | >128 | >128 | >128 | >128 | >128 | >128 | >128 | >128 | >128 |
| K. pneu. | 4005 | <0.25 | 1.0 | 8.0 | 16 | 1.0 | 16 | >128 | >128 | 16 | 32 | >128 | >128 | 8.0 | 16 | 16 | 64 |
| Ps. aer. | 5160[b] | >128 | >128 | >128 | >128 | >128 | >128 | >128 | >128 | >128 | >128 | >128 | >128 | >128 | >128 | >128 | >128 |
| Ps. aer. | 2835 | >128 | >128 | >128 | >128 | >128 | >128 | >128 | >128 | >128 | >128 | >128 | >128 | >128 | >128 | >128 | >128 |

[a] Abbreviations: St. aur. = Staphylococcus aureus; Str. fae. = Streptococcus faecalis; Pr. mir. = Proteus mirabilis;
E. coli = Escherichia coli; K. pneu. = Klebsiella pneumoniae; Ps. aer. = Pseudomonas aeruginosa

[b] Norfloxacin-resistant strain

## Claims

1. The use of a compound having the formula:

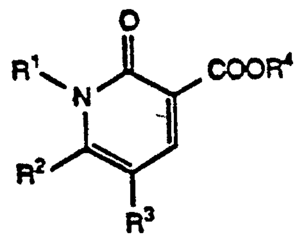

wherein $R^1$ is:
a) hydrogen;
b) $C_1$-$C_6$ alkyl;
c) $C_1$-$C_4$ haloalkyl;
d) $C_2$-$C_6$ alkenyl;
e) $C_4$-$C_{11}$ (cycloalkyl)alkyl; or
f) $C_7$-$C_{14}$ aralkyl;
$R^2$ and $R^3$ are independently:
a) aryl having 6, 10, or 14 nuclear ring carbon atoms or said aryl substituted with one or more substituents selected from the group consisting of:
i) halogen;
ii) hydroxy;
iii) $C_1$-$C_6$ alkyl;
iv) $C_1$-$C_6$ alkoxy;
v)

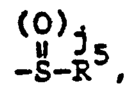

wherein $R^5$ is $C_1$-$C_6$ alkyl, and wherein j is 0, 1, or 2;
vi)

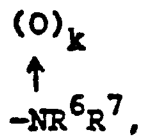

or a pharmaceutically acceptable acid addition salt thereof, wherein $R^6$ and $R^7$ is $C_1$-$C_6$ alkyl, and wherein k is 0 or 1;
vii)

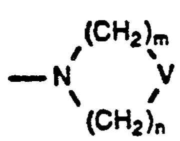

or a pharmaceutically acceptable acid addition salt thereof, wherein V is $CH_2$, O, S, SO, $SO_2$, or $NR^8$ wherein $R^8$ is hydrogen or $C_1$-$C_6$ alkyl, and wherein m and n are independently integers of from 1 to 3;
viii) $-O(CH_2)_pNR^9R^{10}$, or a pharmaceutically acceptable acid addition salt thereof, wherein $R^9$ and $R^{10}$ are

independently $C_1$-$C_6$ alkyl or taken together are $C_2$-$C_6$ alkylene, and wherein p is an integer of from 1 to 6; and

ix) -Y-$Ar^1$, wherein Y is absent or is $CH_2$, O, $-OCH_2-$, or S, and wherein $Ar^1$ is aryl having 6, 10 or 14 nuclear ring carbon atoms or is heteroaryl having 5 or 6 nuclear ring atoms of which at least one nuclear ring atom is O, S, or N; or

b) heteroaryl having 5 or 6 nuclear ring atoms of which at least one nuclear ring atom is O, S, or N, or said heteroaryl substituted with one or more substituents selected from the group consisting of:

i) halogen;

ii) hydroxy;

iii) $C_1$-$C_6$ alkyl;

iv) $C_1$-$C_6$ alkoxy;

v)

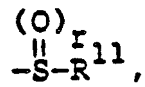

wherein $R^{11}$ is $C_1$-$C_6$ alkyl, and wherein r is 0, 1, or 2;

vi)

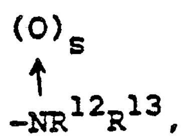

or a pharmaceutically acceptable acid addition salt thereof, wherein $R^{12}$ and $R^{13}$ are independently hydrogen or $C_1$-$C_6$ alkyl, and wherein s is 0 or 1;

vii)

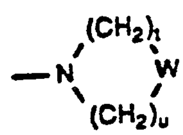

or a pharmaceutically acceptable acid addition salt thereof, wherein W is $CH_2$, O, S, SO, $SO_2$, or $NR^{14}$, wherein $R^{14}$ is hydrogen or $C_1$-$C_6$ alkyl, and wherein t and u are independently integers of from 1 to 3; and

viii) -Z-$Ar^2$, wherein Z is absent or is $CH_2$, O, $-OCH_2-$, or S, and wherein $Ar^2$ is aryl having 6, 10, or 14 nuclear ring carbon atoms or is heteroaryl having 5 or 6 nuclear ring atoms of which at least one nuclear ring atom is O, S, or N; and

$R^4$ is:

a) hydrogen;

b) $C_1$-$C_6$ alkyl;

c) a pharmaceutically acceptable cation; or

d) a prodrug ester group;

for the preparation of a medicament useful for treating bacterial infections.

2. The use according to Claim 1 wherein $R^4$ is hydrogen.

3. The use according to Claim 1 wherein the compound has the formula:

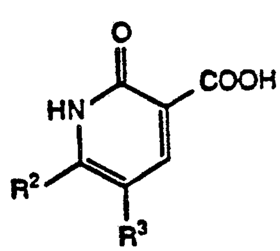

wherein $R^2$ and $R^3$ are independently:

a) aryl having 6, 10, or 14 nuclear ring carbon atoms or said aryl substituted with one or more substituents selected from the group consisting of:

i) halogen;

ii) hydroxy;

iii) $C_1$-$C_6$ alkyl;

iv) $C_1$-$C_6$ alkoxy;

v)

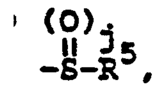

wherein $R^5$ is $C_1$-$C_6$ alkyl, and wherein j is 0, 1, or 2;

vi)

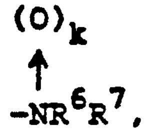

or a pharmaceutically acceptable acid addition salt thereof, wherein $R^6$ and $R^7$ are independently hydrogen or $C_1$-$C_6$ alkyl, and wherein k is 0 or 1;

vii)

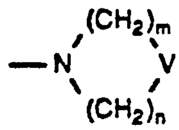

or a pharmaceutically acceptable acid addition salt thereof, wherein V is $CH_2$, O, S, SO, $SO_2$, or $NR^8$, wherein $R^8$ is hydrogen or $C_1$-$C_6$ alkyl, and wherein m and n are independently integers of from 1 to 3;

viii) $-O(CH_2)_pNR^9R^{10}$, or a pharmaceutically acceptable acid addition salt thereof, wherein $R^9$ and $R^{10}$ are independently $C_1$-$C_6$ alkyl or taken together are $C_2$-$C_6$ alkylene, and wherein p is an integer of from 1 to 6; and

ix) $-Y-Ar^1$, wherein Y is absent or is $CH_2$, O, $-OCH_2-$, or S, and wherein $Ar^1$ is aryl having 6, 10 or 14 nuclear ring carbon atoms or is heteroaryl having 5 or 6 nuclear ring atoms of which at least one nuclear ring atom is O, S, or N; or

b) heteroaryl having 5 or 6 nuclear ring atoms of which at least one nuclear ring atom is O, S, or N, or said heteroaryl substituted with one or more substituents selected from the group consisting of:

i) halogen;

ii) hydroxy;

iii) $C_1$-$C_6$ alkyl;

iv) $C_1$-$C_6$ alkoxy;

v)

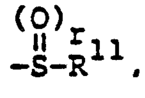

wherein $R^{11}$ is $C_1$-$C_6$ alkyl, and wherein r is 0, 1, or 2;

vi)

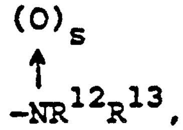

or a pharmaceutically acceptable acid addition salt thereof, wherein $R^{12}$ and $R^{13}$ are independently hydrogen or $C_1$-$C_6$ alkyl, and wherein s is 0 or 1;
vii)

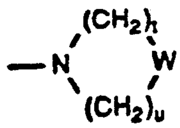

or a pharmaceutically acceptable acid addition salt thereof, wherein W is $CH_2$, O, S, SO, $SO_2$, or $NR^{14}$, wherein $R^{14}$ is hydrogen or $C_1$-$C_6$ alkyl, and wherein t and u are independently integers of from 1 to 3;
viii) -Z-$Ar^2$, wherein Z is absent or is $CH_2$, O, -$OCH_2$-, or S, and wherein $Ar^2$ is aryl having 6, 10, or 14 nuclear ring carbon atoms or is heteroaryl having 5 or 6 nuclear ring atoms of which at least one nuclear ring atom is O, S, or N.

4. The use according to Claim 3 wherein the compound has the formula:

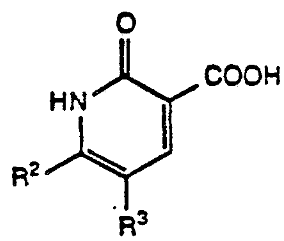

wherein $R^2$ is:
a) phenyl substituted with one or more substituents selected from the group consisting of:
i) halogen;
ii) hydroxy;
iii) $C_1$-$C_6$ alkyl;
iv) $C_1$-$C_6$ alkoxy;
v)

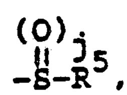

wherein $R^5$ is $C_1$-$C_6$ alkyl, and wherein j is 0, 1, or 2;
vi)

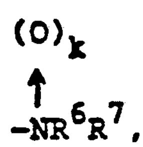

or a pharmaceutically acceptable acid addition salt thereof, wherein $R^6$ and $R^7$ are independently $C_1$-$C_6$ alkyl, and wherein k is 0 or 1;
vii)

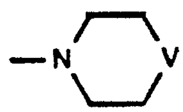

or a pharmaceutically acceptable acid addition salt thereof, wherein V is $CH_2O$, or $NR^8$, wherein $R^8$ is $C_1$-$C_6$ alkyl;
viii) -$OCH_2CH_2NR^9R^{10}$, or a pharmaceutically acceptable acid addition salt thereof, wherein $R^9$ and $R^{10}$ are independently $C_1$-$C_6$ alkyl; and
ix) -Y-$Ar^1$, wherein Y is absent or is $CH_2$, -$OCH_2$-, or S, and wherein $Ar^1$ is phenyl or pyridinyl; or
b) thienyl substituted with thienyl; and
$R^3$ is:
a) phenyl substituted with one or more substituents selected from the group consisting of:
i) halogen;
ii) hydroxy;
iii) $C_1$-$C_6$ alkoxy; and
iv) benzyloxy; or
b) pyridinyl.

5. The use according to Claim 4 wherein the compound has the formula:

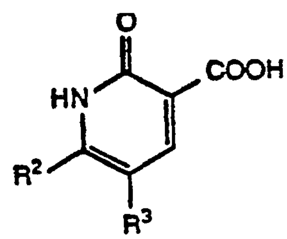

wherein $R^2$ is:
a) phenyl substituted with one or more substituents selected from the group consisting of:
i) fluorine;
ii) methoxy;
iii)

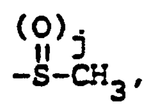

wherein j is 0, 1, or 2;
iv)

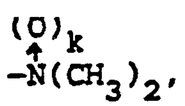

or a pharmaceutically acceptable acid addition salt thereof, wherein k is 0 or 1;
v)

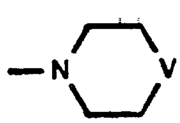

or a pharmaceutically acceptable acid addition salt thereof, wherein V is $CH_2$, O, or $NCH_3$;
vi) -$OCH_2CH_2N(CH_2CH_3)_2$, or a pharmaceutically acceptable acid addition salt thereof, and
vii) pyridinylthio; or
b) thienyl substituted with thienyl; and

$R^3$ is:

a) phenyl substituted with one or more substituents selected from the group consisting of:

i) fluorine

ii) hydroxy;

iii) methoxy; and

iv) benzyloxy; or

b) pyridinyl.

6. The use according to Claim 1 wherein the compound has the formula:

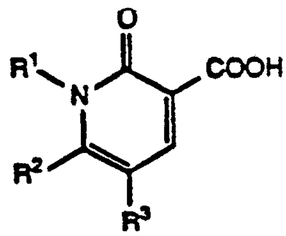

wherein $R^1$ is:

a) $C_1$-$C_6$ alkyl;

b) $C_1$-$C_4$ haloalkyl;

c) $C_2$-$C_6$ alkenyl;

d) $C_4$-$C_{11}$ (cycloalkyl)alkyl; or

e) $C_7$-$C_{14}$ aralkyl;

$R^2$ and $R^3$ are independently:

a) aryl having 6, 10, or 14 nuclear ring carbon atoms or said aryl substituted with one or more substituents selected from the group consisting of:

i) halogen;

ii) hydroxy;

iii) $C_1$-$C_6$ alkyl;

iv) $C_1$-$C_6$ alkoxy;

v)

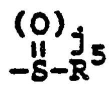,

wherein $R^5$ is $C_1$-$C_6$ alkyl, and wherein j is 0, 1, or 2;

vi)

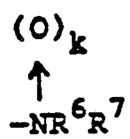,

or a pharmaceutically acceptable acid addition salt thereof, wherein $R^6$ and $R^7$ are independently hydrogen or $C_1$-$C_6$ alkyl, and wherein k is 0 or 1;

vii)

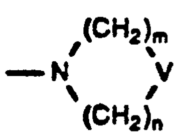

or a pharmaceutically acceptable acid addition salt thereof, wherein V is $CH_2$, O, S, SO, $SO_2$, or $NR^8$, wherein $R^8$ is hydrogen or $C_1$-$C_6$ alkyl, and wherein m and n are independently integers of from 1 to 3;

viii) $-O(CH_2)_pNR^9R^{10}$, or a pharmaceutically acceptable acid addition salt thereof, wherein $R^9$ and $R^{10}$ are independently $C_1$-$C_6$ alkyl or taken together are $C_2$-$C_6$ alkylene, and wherein p is an integer of from 1 to 6; and

ix) -Y-Ar', wherein Y is absent or is $CH_2$, O, $-OCH_2-$, or S, and wherein $Ar^1$ is aryl having 6, 10 or 14 nuclear ring carbon atoms or is heteroaryl having 5 or 6 nuclear ring atoms of which at least one nuclear ring atom is O, S, or N; or

b) heteroaryl having 5 or 6 nuclear ring atoms of which at least one nuclear ring atom is O, S, or N, or said heteroaryl substituted with one or more substituents selected from the group consisting of:

i) halogen;

ii) hydroxy;

iii) $C_1$-$C_6$ alkyl;

iv) $C_1$-$C_6$ alkoxy;

v)

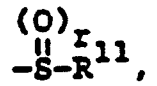

wherein $R^{11}$ is $C_1$-$C_6$ alkyl, and wherein r is 0, 1, or 2;

vi)

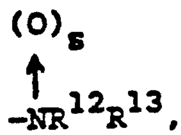

or a pharmaceutically acceptable acid addition salt thereof, wherein $R^{12}$ and $R^{13}$ are independently hydrogen or $C_1$-$C_6$ alkyl, and wherein S is 0 or 1;

vii)

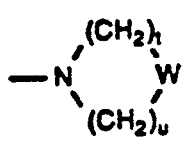

or a pharmaceutically acceptable acid addition salt thereof, wherein W is $CH_2$, O, S, SO, $SO_2$, or $NR^{14}$, wherein $R^{14}$ is hydrogen or $C_1$-$C_6$ alkyl, and wherein t and u are independently integers of from 1 to 3; and

viii) $-Z-Ar^2$, wherein Z is absent or is $CH_2$, O, $-OCH_2-$, or S, and wherein $Ar^2$ is aryl having 6, 10, or 14 nuclear ring carbon atoms or is heteroaryl having 5 or 6 nuclear ring atoms of which at least one nuclear ring atom is O, S, or N.

7. The use according to Claim 6 wherein the compound has the formula:

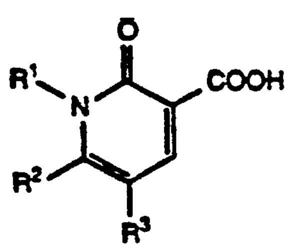

wherein $R^1$ is:

a) $C_1$-$C_6$ alkyl;

b) allyl;

c) cyclopropylmethyl; or

d) benzyl;

$R^2$ is phenyl substituted with dimethylamino or a pharmaceutically acceptable acid addition salt thereof; and $R^3$ is phenyl substituted with halogen or hydroxy.

8. A pharmaceutical composition useful for the treatment of bacterial infections comprising a pharmaceutically acceptable amount of at least one compound having the formula:

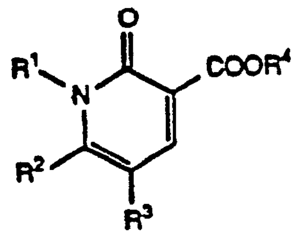

together with one or more pharmaceutically acceptable carriers;
wherein $R^1$ is:
a) hydrogen;
b) $C_1$-$C_6$ alkyl;
c) $C_1$-$C_4$ haloalkyl;
d) $C_2$-$C_6$ alkenyl;
e) $C_4$-$C_{11}$ (cycloalkyl)alkyl; or
f) $C_7$-$C_{14}$ aralkyl;
$R^2$ and $R^3$ are independently:
a) aryl having 6, 10, or 14 nuclear ring carbon atoms or said aryl substituted with one or more substituents selected from the group consisting of:
i) halogen;
ii) hydroxy;
iii) $C_1$-$C_6$ alkyl;
iv) $C_1$-$C_6$ alkoxy;
v)

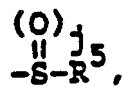

wherein $R^5$ is $C_1$-$C_6$ alkyl, and wherein j is 0, 1, or 2;
vi)

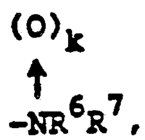

or a pharmaceutically acceptable acid addition salt thereof, wherein $R^6$ and $R^7$ is $C_1$-$C_6$ alkyl, and wherein k is 0 or 1;
vii)

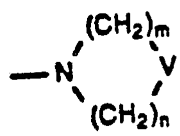

or a pharmaceutically acceptable acid addition salt thereof, wherein V is $CH_2$, O, S, SO, $SO_2$, or $NR^8$, wherein $R^8$ is hydrogen or $C_1$-$C_6$ alkyl, and wherein m and n are independently integers of from 1 to 3;
viii) $-O(CH_2)_pNR^9R^{10}$, or a pharmaceutically acceptable acid addition salt thereof, wherein $R^9$ and $R^{10}$ are independently $C_1$-$C_6$ alkyl or taken together are $C_2$-$C_6$ alkylene, and wherein p is an integer of from 1 to 6; and

ix) -Y-$Ar^1$, wherein Y is absent or is $CH_2$, O, -$OCH_2$-, or S, and wherein $Ar^1$ is aryl having 6, 10 or 14 nuclear ring carbon atoms or is heteroaryl hving 5 or 6 nuclear ring atoms of which at least one nuclear ring atom is O, S, or N; or

b) heteroaryl having 5 or 6 nuclear ring atoms of which at least one nuclear ring atom is O, S, or N, or said heteroaryl substituted with one or more substituents selected from the group consisting of:

i) halogen;

ii) hydroxy;

iii) $C_1$-$C_6$ alkyl;

iv) $C_1$-$C_6$ alkoxy;

v)

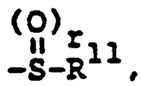

wherein $R^{11}$ is $C_1$-$C_6$ alkyl, and wherein r is 0, 1, or 2;

vi)

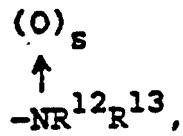

or a pharmaceutically acceptable acid addition salt thereof, wherein $R^{12}$ and $R^{13}$ are independently hydrogen or $C_1$-$C_6$ alkyl, and wherein s is 0 or 1;

vii)

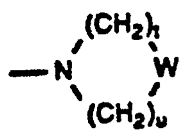

or a pharmaceutically acceptable acid addition salt thereof, wherein W is $CH_2$, O, S, SO, $SO_2$, or $NR^{14}$, wherein $R^{14}$ is hydrogen or $C_1$-$C_6$ alkyl, and wherein t and u are independently integers of from 1 to 3; and

viii) -Z-$Ar^2$, wherein Z is absent or is $CH_2$, O, -$OCH_2$-, or S, and wherein $Ar^2$ is aryl having 6, 10, or 14 nuclear ring carbon atoms or is heteroaryl having to or 6 nuclear ring atoms of which at least one nuclear ring atom is O, S, or N;and

$R^4$ is:

a) hydrogen;

b) $C_1$-$C_6$ alkyl;

c) a pharmaceutically acceptable cation; or

d) a prodrug ester group.

9. A pharmaceutical composition according to Claim 8 wherein said compound is selected from the group consisting of:

6-[4-(dimethylamino)phenyl]-1,2-dihydro-5-(4-methoxyphenyl)-2-oxo-3-pyridinecarboxylic acid;

6-[4-(dimethylamino)phenyl]-1,2-dihydro-5-(4-hydroxyphenyl)-2-oxo-3-pyridinecarboxylic acid;

5-(4-fluorophenyl)-1,2-dihydro-6-(4-methoxyphenyl)-2-oxo-3-pyridinecarboxylic acid;

6-[4-(dimethylamino)phenyl]-5-(4-fluorophenyl)-1,2-dihydro-2-oxo-3-pyridinecarboxylic acid;

6-[4-(dimethylamino)-3-fluorophenyl]-1,2-dihydro-5-(4-hydroxyphenyl)-2-oxo-3-pyridinecarboxylic acid;

6-[4-(dimethylamino)-2-fluorophenyl]-1,2-dihydro-5-(4-hydroxyphenyl)-2-oxo-3-pyridinecarboxylic acid;

5-(4-fluorophenyl)-1,2-dihydro-6-[4-(methylthio)phenyl]-2-oxo-3-pyridinecarboxylic acid;

1,2-dihydro-5-(4-hydroxyphenyl)-2-oxo-6-[4-(2-pyridinylthio)phenyl]-3-pyridinecarboxylic acid;

1,2-dihydro-6-(4-methoxyphenyl)-2-oxo-5-[4-(phenylmethoxy)phenyl]-3-pyridinecarboxylic acid;

1,2-dihydro-5-(4-hydroxyphenyl)-2-oxo-6-[5-(2-thienyl)-2-thienyl]-3-pyridinecarboxylic acid;

6-[4-[2-(diethylamino)ethoxyl]phenyl]-1,2-dihydro-2-oxo-5-[4-(phenylmethoxy)phenyl]-3-pyridinecarboxylic

acid;
6-[4-(dimethylamino)phenyl]-1,2-dihydro-2-oxo-5-(4-pyridinyl)-3-pyridinecarboxylic acid;
5-(4-fluorophenyl)-1,2-dihydro-2-oxo-6-(4-piperidinophenyl)-3-pyridinecarboxylic acid;
5-(4-fluorophenyl)-1,2-dihydro-6-(4-morpholinophenyl)-2-oxo-3-pyridinecarboxylic acid;
5-(4-fluorophenyl)-1,2-dihydro-6-[4-(4-methyl-1-piperazinyl)phenyl]-2-oxo-3-pyridinecarboxylic acid;
1,2-dihydro-5-(4-hydroxyphenyl)-6-(4-methoxyphenyl)-2-oxo-3-pyridinecarboxylic acid;
6-[4-[2-(diethylamino)ethoxy]phenyl]-1,2-dihydro-5-(4-hydroxyphenyl)-2-oxo-3-pyridinecarboxylic acid;
5-(4-fluorophenyl)-1,2-dihydro-6-[4-(methylsulfonyl)phenyl]-2-oxo-3-pyridinecarboxylic acid;
5-(4-fluorophenyl)-1,2-dihydro-6-[4-(methylsulfinyl)phenyl]-2-oxo-3-pyridinecarboxylic acid;
6-[4-(dimethylamino)phenyl]-5-(4-fluorophenyl)-1,2-dihydro-2-oxo-3-pyridinecarboxylic acid N-oxide;
6-[4-(dimethylamino)phenyl]-1,2-dihydro-5-(4-hydroxyphenyl)-2-oxo-3-pyridinecarboxylic acid N-oxide;
6-[4-(dimethylamino)phenyl]-1,2-dihydro-5-(4-hydroxyphenyl)-1-methyl-2-oxo-3-pyridinecarboxylic acid;
6-[4-(dimethylamino)phenyl]-5-(4-fluorophenyl)-1,2-dihydro-1-methyl-2-oxo-3-pyridinecarboxylic acid;
6-[4-(dimethylamino)phenyl]-5-(4-fluorophenyl)-1,2-dihydro-2-oxo-1-(2-propenyl)-3-pyridinecarboxylic acid;
1-(cyclopropylmethyl)-6-[4-(dimethylamino)phenyl]-5-(4-fluorophenyl)-1,2-dihydro-2-oxo-3-pyridinecarboxylic acid; and
6-[4-(dimethylamino)phenyl]-5-(4-fluorophenyl)-1,2-dihydro-2-oxo-1-(phenylmethyl)-3-pyridinecarboxylic acid.

10. The use of a pharmaceutical composition of Claim 9 for the preparation of a medicament useful for treating bacterial infections.